# EUROPEAN PATENT SPECIFICATION

(11) **EP 3 833 345 B1**
(45) Date of publication and mention of the grant of the patent: **17.07.2024**
(21) Application number: 19756327.3
(22) Date of filing: 05.08.2019
(51) Int. Cl.: A61K 31/427, A61P 35/00

(54) **SMALL MOLECULE-INHIBITORS OF 6-PHOSPHOFRUCTO-1-KINASE FOR REDUCING LACTATE GENERATION BY CANCER CELLS**
KLEINE MOLEKÜLEN ALS HEMMER DER PHOSPHOFRUCTOKINASE ZUR UNTERDRÜCKUNG DER LAKTATPRODUKTION IN KREBSZELLEN
DES MOLÉCULES DE PETITE TAILLE INHIBITRICES DE LA PHOSPHOFRUCTOKINASE POUR LA RÉDUCTION DE LA PRODUCTION DE LACTATE PAR LES CELLULES CANCÉREUSES

(30) Priority: 07.08.2018 EP 18187667
(43) Date of publication of application: 16.06.2021
(73) Proprietor: Kemijski Institut, 1001 Ljubljana (SI)
(72) Inventor: LEGISA, Matic, 1215 Medvode (SI); LESNIK, Samo, 2322 Majsperk (SI); KONC, Janez, 1000 Ljubljana (SI); SOLMAJER, Tomaz, 1000 Ljubljana (SI); VODOPIVEC, Tina, 6230 Postojna (SI); CAMERNIK, Katja, 1351 Brezovica (SI); K POTOKAR, Urska, 1000 Ljubljana (SI); KAVCIC, Luka, 4220 Skofja Loka (SI)
(74) Representative: Patentni Biro AF d.o.o.
(86) International application number: PCT/EP2019/071059
(87) International publication number: WO 2020/030613

(56) References cited:
- WO-A1-2017/095751
- DATABASE BIOSIS [online] BIOSCIENCES INFORMATION SERVICE, PHILADELPHIA, PA, US; 16 October 2017 (2017-10-16), LEE JONG-HO ET AL: "Stabilization of phosphofructokinase 1 platelet isoform by AKT promotes tumorigenesis", Database accession no. PREV201800009126
- NATURE COMMUNICATIONS, vol. 8, 16 October 2017 (2017-10-16), pages Article No.: 949, ISSN: 2041-1723, DOI: 10.1038/S41467-017-00906-9
- DATABASE PUBCHEM COMPOUND [online] NCBI; 13 November 2007 (2007-11-13), ANONYMOUS: "2-[[5-(1,3-benzodioxol-5-yl)-1,3,4-oxadiazol-2-yl]sulfanyl]-N-(1,2-oxazol-3-yl)acetamide", XP002795151, Database accession no. CID17466824
- DATABASE REGISTRY [online] CHEMICAL ABSTRACTS SERVICE, COLUMBUS, OHIO, US; 18 September 2009 (2009-09-18), "3-methoxy-6-[3-[1-[(5-methyl-1,2,4-oxadiazol-3-yl)methyl]-1H-pyrazol-3-yl]phenyl]-pyridazine", XP002795152, Database accession no. 1185676-71-4
- DATABASE PUBCHEM COMPOUND [online] NCBI; 25 November 2010 (2010-11-25), ANONYMOUS: "N-[5-(methanesulfonamido)-1,3,4-thiadiazol-2-yl]-6,7,8,9-tetrahydro-5H-carbazole-3-carboxamide", XP002795153, Database accession no. CID47049402
- DATABASE PUBCHEM COMPUND [online] NCBI; 13 November 2007 (2007-11-13), ANONYMOUS: "3-(4-chlorophenyl)sulfonyl-N-[5-(1,2-oxazol-5-yl)-1,3,4-oxadiazol-2-yl]propanamide", XP002795154, Database accession no. CID16861359
- DATABASE PUBCHEM COMPOUND [online] NCBI; 13 July 2005 (2005-07-13), ANONYMOUS: "4-[3-(5-amino-1,3,4-oxadiazol-2-yl)-1,2-oxazol-5-yl]phenol", XP002795155, Database accession no. CID2026535
- DATABASE PUBCHEM COMPUND [online] NCBI; 30 May 2009 (2009-05-30), ANONYMOUS: "(3R)-N-[5-(1,2-oxazol-5-yl)-1,3,4-oxadiazol-2-yl]-2,3-dihydro-1,4-benzodioxine-3-carboxamide", XP002795156, Database accession no. CID42477491
- DATABASE PUBCHEM COMPOUND [online] NCBI; 13 November 2007 (2007-11-13), ANONYMOUS: "3-(benzenesulfony)-N-[5-(1,2-oxazol-5-yl)-1,3,4-oxadiazol-2-yl]propanamide", XP002795157, Database accession no. CID16861348
- LEE JONG-HO ET AL: "Stabilization of phosphofructokinase 1 platelet isoform by AKT promotes tumorigenesis", NATURE COMMUNICATIONS, vol. 8, 16 October 2017 (2017-10-16), XP002795158, ISSN: 2041-1723

## Description

### Field of the invention

The present invention belongs to the field of compounds having therapeutically relevant activity, more precisely to the field of compounds targeting the glycolytic pathway. The invention relates to seven compounds that dock to the ATP binding site of the cancer-specific 6-phosphofructo-1-kinase (PFK1), inhibit its activity and significantly decrease lactate generation by the cancer cells. The targeted enzyme is believed to be the pivotal factor of deregulated glycolytic flux in cancer cells and concomitantly causes abundant lactate production (Warburg effect) that enables immune escape by cancer cells.

### Background of the invention and the technical problem

Cancer is a disease in which cells undergo uncontrolled proliferation, resulting in the formation of a mass of cells. The deviant energetic metabolism of cancer cells is characterized by the consumption of larger amounts of glucose compared to the normal cells and the conversion of the majority of glucose into lactic acid. This occurs even in the presence of ample oxygen; the process is known as the "Warburg effect" or aerobic glycolysis. Higher rate of glycolysis and redundant cytosolic NADH generation is therefore at the root of tumour formation and growth. In cells of all organisms, maintenance of redox homeostasis is of utmost importance. In cancer cells surplus of NADH is solved by its rapid re-oxidation and concomitant reduction of pyruvate to lactic acid. Up to 90% of total glucose metabolism is accounted for lactic acid formation, therefore, it is necessary for the cells to excrete large amounts of lactate (DeBarardinis et al, 2007; doi:10.1016/j.bbapap.2018.03.005). Extracellular accumulation of protons released form lactic acid leads to acidification of tumour microenvironment creating pH gradient between extracellular pHₑ (6,6-6,9) and intracellular pHᵢ (7,2-7,5) that supports malignant phenotype (Webb et al, 2011; doi:10.1038/nrc3110). Lactic acid is a potent inhibitor of function and survival of T cells, NK lymphocytes and macrophages, consequently, it is believed to be responsible for enabling cancer cells to escape the immune system (Brand et al., 2016; doi:10.1016/j.cmet.2016.08.011). It also triggers angiogenesis and stimulates tumour cells migration that induces metastatic dissemination of tumours (Hirschhaeuser et al., doi:10.1158/0008-5472.CAN-11-1457). Consequently, compounds that could prevent lactate excretion might be of extreme importance for the success of cancer treatment.

Simultaneously with the progress in understandings of cancer metabolism, a number of new possible drug targets were identified. Unfortunately, a relatively low number of metabolic inhibitors has been developed so far that are in regular use for cancer treatment. It is worth noting that there is a lot of uniformity between the metabolism of malignant cells and that of highly proliferating normal cells, therefore such inhibitors can cause significant side effects. The most promising metabolic targets for cancer therapy have been recently nicely discussed by Galluzzi et al. (2013; doi:10.1038/nrd4145). In the review, the compounds which treatment is in the phase of preclinical trials (WZB117, BPTES), some that have promoted to clinical development but were discontinued (3-bromopyruvate, Methyl-jasmonate, Thallion), and few with clinical experience (Metformin, Phenformin, Dichloroacetate) are listed. Among them, there are just some that target glycolytic enzymes such as cancer specific hexokinase (HK2) (3-bromopyruvate) and pyruvate kinase (PKM2) (Thallion) and are in the advanced level of anti-cancer trials (clinical studies). Some others are in the initial stage of testing.

Alternatively, there have been several attempts in the past to inhibit lactate generation in the cancer cells. The most promising target seemed to be lactate dehydrogenases that catalyse the reduction of pyruvate to lactate on the expense of NADH. Deletion of the *LDHA* gene resulted in inhibited growth of several tumorigenic cell lines (Shim et al., 1997; PNAS 94:6658-63) and reduced growth of transplanted breast tumours (doi: 10.1016/J.CCR.2006.04.023). Further a small molecule FX11 that acts as an inhibitor of LDHA impaired growth of human pancreatic cancer and lymphoma xenografts. However, inactivation of LDHA led to the enhanced production of ROS, which is aggravated by hypoxia, and drives cancer progression (Le et al., doi:10.1073/pnas.0914433107). Besides several other LDHA inhibitors have been tested for their anticancer activity. Document WO2016109559 discloses gossypol, a natural product of cottonseeds, as a nonselective inhibitor of LDH that blocks the binding of NADH, with a Kᵢ for LDHA and LDHB of 1.9 and 1.4 µM respectively.

Additional promising targets for lactate excretion are the lactate transporters (MCTs) that are critical regulators of intracellular lactate and pH. More small molecules that act as inhibitors of LDHs and MCTs have the potential to be developed into cancer therapeutics and have been described and listed by Doherty and Cleveland (doi:10.1 172/JCI69741).

In addition, dichloroacetate (DCA) is in clinical use for the treatment of lactic acidosis (Michelakis et al., doi:10.1038/sj.bjc.6604554). It is well tolerated by patients with glioblastoma and provokes profound mitochondrial defects in cancer cells. DCA acts as an inhibitor of pyruvate dehydrogenase kinase (PDK) that regulates the flux of carbohydrates (pyruvate) into the mitochondria. In the presence of active PDK, pyruvate dehydrogenase (PDH) is inhibited, limiting the entry of pyruvate into the mitochondria that consequently promotes lactate formation and excretion. On the other hand, if PDK is inhibited, PDH remains active and the majority of pyruvate enters mitochondria, therefore, lactate formation is reduced. DCA has been studied to alleviate the symptoms or the haemodynamic consequences of the lactic acidosis also in other diseases: complicating severe malaria, congestive heart failure, burns, cirrhosis, liver transplantation and congenital mitochondrial diseases. It might be also beneficial for the treatment of human cancers, however, lactate in cancers is generated mostly due to the need for re-oxidation of abundant NADH, which can't be prevented by this drug.

However, the most prominent change of an allosteric glycolytic enzyme in cancer cells seems to be a posttranslational modification of PFK1 (Smerc et al., doi:10.1371/journal.pone.0019645). This enzyme is the key regulatory enzyme of glycolysis that catalyses the phosphorylation of fructose-6-phosphate to fructose-1,6-bisphosphate (FBP) using Mg-ATP as a phosphoryl donor. It is stimulated by fructo-2,6-bisphosphate (F2,6BP), ADP/AMP, and ammonium ions, whereas citrate and ATP act as strong inhibitors (Dunaway et al., Biochem J. 251: 677-83).

Three isoforms of PFK1 enzyme are known, PFK-M, PFK-L and PFK-P. Their expression in cancer cells and normal cells differs significantly. We were first to describe a posttranslational modification of human 6-phosphofructo-1-kinase (PFK1) in the cancer cells. Namely, the native muscle type 85 kDa form (PFK-M) can be cleaved by specific protease forming a 45 kDa shorter fragment (Smerc et al. doi: 10.1371/journal.pone.0019645; Andrejc et al., doi:10.1016/j.bbapap.2018.03.005). Newly produced fragments retain activity but have altered kinetic characteristics. The truncated enzyme is resistant to feedback inhibition by citrate and ATP, whereas some effectors, such as F2,6BP, increase enzyme's activity to a level higher than that of the native enzyme. Similar fragments have also been found for P and L isoforms. Such a significant change of an allosteric enzyme leads to deregulated glycolytic flux and increased cytosolic NADH production. However, in the cells of all organisms, maintenance of redox homeostasis is of utmost importance. In cancer cells, the abundance of NADH is solved by its rapid re-oxidation and concomitant reduction of pyruvic to lactic acid.

Reduced control of the glycolytic flux at the level of PFK1 in the cancer cells has been confirmed also by control analysis in two fast-growing cell types; human HeLa cells and rodent As-30D cells while kinetic analysis of PFK1 showed low sensitivity towards its allosteric inhibitors citrate and ATP (Marin-Hernandez et al, FEBS J 273:1975-88; and Marin-Hernandez et al., doi:10.1016/j.bbabio.2010.11.006).

For curing cancers, it would be of utmost importance to minimize glycolytic flux and consequently prevent lactate generation and excretion. Thus, the aim of the invention is to provide compounds that interfere with highly active cancer-specific forms of PFKs to reduce their activities to the level of the native PFKs enzymes found in the normal cells. Partial inhibition of modified cancer-specific PFKs results in decreased glycolytic flux and NADH formation so that redundant NADH re-oxidation is not needed and consequently lactate is not generated anymore. Such treatment doesn't interfere with other cellular mechanisms, it is not cytotoxic, while the possible side effects are minimal.

### State of the art

PFK has been proposed as a target for anti-cancer drugs several times, but only a relatively low number of PFK inhibitors have been discovered and studied. Among them, only compounds affecting activities of native PFK1 iso-enzymes were reported. However, there are no reports of compounds that would target specific kinetic characteristics of the modified, cancer-specific PFK1s.

The drugs described so far to diminish PFK1 activities are cisplatin (Zhou et al., http://www.ncbi.nlm.nih.gov/pubmed/12067998), clotrimazole (Furtado et al., doi:10.1371/journal.pone.0030462; and doi:10.4172/2161-0444.1000219), etoposide (Zhou et al., 2002, http://www.ncbi.nlm.nih.gov/pubmed/12067998), and taxol (Glass-Marmor et al, doi: 10.1016/s0014-2999(99)00155-7). Taxol induces detachment of PFK from the cytoskeleton and was reported to influence PFK1 activity by reducing the gene expression. However, these substances act by reducing the transcription of not only the *pfk* genes but also other glycolytic genes. For instance, cisplatin that is used for clinical treatment of testicular, ovarian, cervical head and neck, non-small cell lung cancers and relapsed lymphomas, impairs transcription and replication that promotes apoptosis (Zhou et al., http://www.ncbi.nlm.nih.gov/pubmed/12067998). Similarly, the true inhibition of 3-bromopyruvate for HK2 is limited, as this agent has been shown to inhibit additional enzymes that are involved in glycolysis, Including glyceraldehyde-3-phosphate dehydrogenase (GAPDH) and mitochondrial succinate dehydrogenase (Shosan; doi:10.1007/s10863-012-9419-2). Apparently, such compounds likely inhibit bioenergetic metabolism also in the normal cells at the same doses assayed to arrest tumour progression and cause significant side effects. Similar systemic toxicity was observed also with other substances listed above that exhibit multi-target inhibitory effects (Galluzzi et al., doi: 10.1038/nrd4145; Ganapathy-Kanniappan and Geschwind, doi: 10.1186/1476-4598-12-152).

Small-molecule inhibition of 6-phosphofructo-2-kinase/fructose-2,6-bisphosphatase (PFKFB3) proved to be a promising target for reducing enhanced glycolytic flux in the cancer cells. In neoplastic cells, PFKFB3 is the most abundant form among 4 isoenzymes since it is constitutively expressed and is required for the high glycolytic rate. Although it is not a glycolytic enzyme, its product Fructose-2,6-bisphosphate (F-2,6-BP) acts as a strong activator of PFK1. For drug design, PFKFB3 3D-crystallographic structure model was prepared and three characteristic amino acid residues from the substrate-binding site were selected for the virtual screening runs. After examining 13 different compounds for their ability to inhibit recombinant PFKFB3 isoenzyme activity, a single compound 3-(3-pyridinyl)-1-(4-pyridinyl)-2-propen-1-one (3PO) was found to suppress basal enzymatic activity. 3PO decreased the intracellular concentration of F-2,6-BP, suppressed glucose uptake, lowered ATP, NADH, NAD⁺ levels and diminished lactate excretion in tumorigenic Jurkat cells. 3PO also markedly attenuated the proliferation of several other human malignant cell lines (US Patent No. 8,088,385). However, 3PO was found to cause an arrest in the cell cycle progression in Jurkat cells and therefore its pharmacokinetic properties were significantly below those required to justify testing in human subjects. In this respect, 73 derivatives of 3PO were synthesized and screened for inhibition of PFKFB3. One molecule, 1-(4-pyridinyl)-3-(2-quinolinyl)-2-propen-1-one (PFK15) was selected for further pre-clinical evaluation. Positive initial results promoted advanced phase 1 clinical trials of its efficacy in advanced cancer patients in 2013 as given in US Patent No. 8,088,385. Later, in 2014, another 3PO derivative, PFK158 was discovered, that is more potent than 3PO, has improved pharmacokinetic properties for testing in clinical trials and causes -80% growth inhibition in several mouse models and human-derived tumours (http://www.cancerandmetabolism.com/content/2/S1 /P14). It has been shown that inhibition of PFKFB3 using PFK158 inhibitor resulted in reduced glucose uptake, ATP production, lactate release as well as induction of apoptosis in gynaecological cancer cells (Mondal S. et al., doi:10.1002/ijc.31868).

Document WO2017095751 describes some possible inhibitors of the PFK enzyme; however, the proposed inhibitors do not appear to be tested for their in vitro and in vivo activity. Further, they differ from the present invention. Also, the following articles have proposed inhibition of PFK enzyme and its importance in treating cancer:
- Yi et al. (2013) PFK1 Glycosylation Is a Key Regulator of Cancer Cell Growth and Central Metabolic Pathways
- Granchi and Minutolo (2013): Anti-cancer agents counteracting tumour glycolysis
- Hasawi et al. (2014): https://www.croh-online.com/article/S1040-8428(14)00086-9/pdf)
- Prasad et al. (2017): Reduced production and uptake of lactate are essential for the ability of WNT5A signalling to inhibit breast cancer cell migration and invasion
These articles do not propose the same inhibitors as the present invention.

To our knowledge, there was no compound described so far that was designed specifically to inhibit any of the human native PFK1 isoforms or active modified forms, in order to function as an anticancer drug.

### Description of the invention

The essence of the invention are seven compounds, which bind to the ATP binding site on human 6-phosphofructo-1-kinase (PFK1), namely:
- Inhibitor No. 3 (2-[(5-benzo[1 ,3]dioxol-5-yl-1,3,4-oxadiazol-2-yl)sulfanyl]-N-isoxazol-3-yl-acetamide),
- Inhibitor No. 9 (3-methoxy-6-(3-{1-[(5-methyl-1,2,4-oxadiazol-3-yl)methyl]-1H-pyrazol-3-yl}phenyl)pyridazine),
- Inhibitor No. 23 N-[5-(methanesulfonamido)-1,3,4-thiadiazol-2-yl]-6,7,8,9-tetrahydro-5H-carbazole-3-carboxamide),
- Inhibitor No. 29 (3-(4-chlorophenyl)sulfonyl-N-(5-isoxazol-5-yl-1,3,4-oxadiazol-2-yl)propanamide,
- Inhibitor No. 30 (4-[3-(5-amino-1,3,4-oxadiazol-2-yl)isoxazol-5-yl]phenol),
- Inhibitor No. 31 ((2R)-N-(5-isoxazol-5-yl-1,3,4-oxadiazol-2-yl)-2,3-dihydro-1,4-benzodioxine-2-carboxamide) and
- Inhibitor No. 32 (3-(benzenesulfonyl)-N-(5-isoxazol-5-yl-1,3,4-oxadiazol-2-yl)propanamide).
These seven compounds individually partially inhibit the activities of highly active cancer-specific PFK enzymes, to the level of activities of native PFK iso-enzymes with the aim of reducing glycolytic flux and cytosolic NADH formation, which decreases the necessity of redundant NADH re-oxidation by generating lactate. The compounds in the molar concentrations that prevented lactate generation and excretion had no significant negative effect on growth and/or survival of the treated cancer cells.

For the invention, drug design based on the crystal structure of the human PFK-P isoenzyme published by Webb et al. (2015, doi:10.1038/nature14405) was conducted. The atomic model of the human PFK-P isoenzyme was designed on the basis of the said crystal structure in combination with ATP-Mg²⁺ at 3.1 Å resolution. The model was used to virtually screen the ZINC Drugs NOW database by docking the compounds to the ATP binding site of the enzyme.

Initially, 4.5 million compounds in the database were filtered to exclude expected aggregators and poorly soluble compounds. By using an in-house supercomputer (CROW 16) with approximately 3000 processor cores, 38 compounds were selected and 18 potential PFK-P inhibitors were purchased from Enamine (1-8), Chembridge (9-16) and ChemDiv (17 and 18) for in-depth testing (1^{st} generation of Inhibitors). The said inhibitors are listed in Table 1 below.

**Table 1: tested compounds that are possible PFK-P and PFK-M inhibitors (1^{st} generation)**

| Inhibitor No. | IUPAC name |
|---|---|
| 1 | 5-[[[(3R)-1-(2,3-dihydro-1,4-benzodioxin-6-yl)-5-oxo-pyrrolidine-3-carbonyl]amino]methyl]isoxazole-3 |
| 2 | N-[(1S)-tetrain-1-yl]-3-[3-(4H-1,2,4-triazol-3-yl)-1,2,4-oxadiazol-5-yl]propadamide |
| 3 | 2-[(5-benzo[1,3]dioxol-5-yl-1,3,4-oxadiazol-2-yl)sulfanyl]-N-isoxazol-3-yl-acetamide |
| 4 | 1 -(2,3)-dihydro-1,4-benzodioxine-6-sulfonyl)-N-(5-methyl-1,3,4-oxadiazol-2-yl)piperidine-4-carboxamide |
| 5 | N-(5-methyl-1,3,4-oxadiazol-2-yl)-1-(2-naphthylsulfonyl)piperidine-4-carboxamide |
| 6 | N-(5-methyl-1,3,4-oxadiazol-2-yl)-1-tetralin-2-ylsulfonyl-piperidine-4-carboxamide |
| 7 | 4-(1,3,4-oxadiazol-2-yl)phenyl 2,3-dimethylquinoxaline-6-carboxylate |
| 8 | (5-methyl-1 ,3,4-oxadiazol-2-yl)methyl 3-phenyl-2,1-benzoxazole-5-carboxylate |
| 9 | 3-methoxy-6-(3-{1-[(5-methyl-1,2,4-oxadiazol-3-yl)methyl]-1H-pyrazol-3-yl}phenyl)pyridazine |
| 10 | 4-{[3-(1,3-benzodioxol-5-yl)-1,4,6,7-tetrahydro-5H-pyrazolo[4,3-c]pyridin-5-yl]methyl}-1H-pyrazole-3-carboxylic acid |
| 11 | 3-{[3-(3-fluorophenyl)-6,7-dihydroisoxazolo[4,5-c]pyridin-5(4H)-yl]methyl}-N-methyl-1,2,4-oxadiazole-5-carboxamide |
| 12 | 5-{[3-(1,3-benzodioxol-5-yl)-6,7-dihydroisoxazolo[4,5-c]pyridin-5(4H)-yl]methyl}-1,2,4-oxadiazole-3-carboxamide |
| 13 | 3-{[3-(2-fluorophenyl)-6,7-dihydroisoxazolo[4,5-c]pyridin-5(4H)-yl]methyl}-N-methyl-1,2,4-oxadiazole-5-carboxamide |
| 14 | 3-methyl-6-(3-{1-['5-methyl-1,3,4-oxadiazol-2yl)methyl]-1H-pyrazol-3-yl}phenyl)pyridazine |
| 15 | 5-{[2-(6-methoxy-2-naphthyl)morpholin-4-yl]methyl}-1 ,2,4-oxadiazole-3-carboxamide |
| 16 | 3-{[3-(1,3-benzodioxol-5-yl)-6,7-dihydroisoxazolo[4,5-c]pyridin-5(4H-yl]methyl}-1,2,4-oxadiazole-5-carboxamide |
| 17 | N-(2-hydroxy-5-methyl-phenyl)-4-(3-nitro-1,2,4-triazol-1-yl)butyramide |
| 18 | N-(4-sulfamoylphenyl)-4H-chromeno[3,4-di]isoxazole-8-sulfonamide |

As already mentioned previously, human cells express three types of PFK1s, namely PFK-P, PFK-M and PFK-L. While M and P isoforms have identical ATP binding site, PFK-L's differs from the former in one amino acid. Therefore, a model with a modified ATP binding site (T102R) was used to virtually screen the ZINC Drugs NOW database by the supercomputer (2^{nd} generation of inhibitors). Additional 15 potential PFK-L inhibitors were purchased from Eminem (21-28) and Chembridge (29-36). The said inhibitors are listed in Table 2 below.

**Table 2: tested compounds that are possible PFK-L inhibitors (2^{nd} generation)**

| Inhibitor No. | IUPAC name |
|---|---|
| 21 | 2-[(5-methyl-1,2,4-oxadiazol-3-yl)methylsulfonyl]-N-[(1R)-tetralin-1-yl]acetamide |
| 22 | 2,3-dimethyl-N-[3-(methylsulfonylmethyl)-1,2,4-oxadiazol-5-yl]-1H-indole-5-carboxamide |
| 23 | N-[5-(methanesulfonamido)-1,3,4-thiadiazol-2-yl]-6,7,8,9-tetrahydro-5H-carbazole-3-carboxamide |
| 24 | 2-(5-methylisoxazol-3-yl)oxy-N-[3-(1-methyltetrazol-5-yl)phenyl]acetamide |
| 25 | [3-(1,3,4-oxadiazol-2-yl)phenyl] |
| 26 | N-(5-methyl-1,3,4-oxadiazol-2-yl)-1-(p-tolylsulfonyl)piperidine-4-carboxamide |
| 27 | 2-[3-(4H-1,2,4-triazol-3-ylsulfonyl)propyl]-1,3-benzoxazole |
| 28 | N,4-dimethyl-N-[4-(1,3,4-oxadiazol-2-yl)benzyl]benzenesulfonamide |
| 29 | 3-(4-chlorophenyl)sulfonyl-N-(5-isoxazol-5-yl-1,3,4-oxadiazol-2-yl)propanamide |
| 30 | 4-[3-(5-amino-1,3,4-oxadiazol-2-yl)isoxazol-5-yl]phenol |
| 31 | (2R)-N-(5-isoxazol-5-yl-1,3,4-oxadiazol-2-yl)-2,3-dihydro-1,4-benzodioxine-2-carboxamide |
| 32 | 3-(benzenesulfonyl)-N-(5-isoxazol-5-yl-1,3,4-oxadiazol-2-yl)propanamide |
| 34 | N-(5-isoxazol-5-yl-1,3,4-oxadiazol-2-yl)-2-(2-naphthyl)acetamide |
| 35 | 2-(4-fluorophenyl)sulfonyl-N-(5-isoxazol-5-yl-1,3,4-oxadiazol-2-yl)acetamide |
| 36 | (NZ)-N-[5-(methoxymethyl)-3H-1,3,4-thiadiazol-2-ylidene]-2-[(3R)-2-oxoindolin-3-yl]acetamide |

Initially, all compounds were tested in vitro for inhibition of the purified human native enzyme (85 kDa) and modified cancer-specific shorter fragments (47 kDa) of muscle type PFK-M, followed by measurements of the purified native enzyme (85 kDa) and shorter fragments (70 kDa) of liver type PFK-L isoform. Both recombinant human PFK-M enzymes (Andrejc and Legi 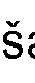 a, doi.org/10.1016/b.bapap.2018.03.005) and both recombinant human PFK-L enzymes were synthesized and isolated from the engineered yeast *S*. *cerevisiae* transformant. By measuring the activities of the enzyme in the presence of different compounds, IC₅₀ values have been determined. Characteristically stronger inhibitory effects were observed in cancer-specific shorter PFK-M or PFK-L fragments (IC₅₀ between 8 to 27 µM) in respect to the native PFK-M and PFK-L enzymes (IC₅₀ between 19 to >50 µM).

Two of compounds, namely Inhibitor No. 3 (2-[(5-benzo[1,3]dioxol-5-yl-1,3,4-oxadiazol-2-yl)sulfanyl]-N-isoxazol-3-yl-acetamide), and Inhibitor No. 9 (3-methoxy-6-(3-{1-[(5-methyl-1,2,4-oxadiazol-3-yl)methyl]-1H-pyrazol-3-yl}phenyl)pyridazine) originate from the 1^{st} generation of screening. First, their inhibitory effect on lactate generation was observed during the growth of acute T-cell leukaemia, Jurkat cells but later similar effects were observed also in colorectal adenocarcinoma Caco2 cells, melanoma Colo 829 cells, and metastatic breast cancer MDA-MB-231 cells.

In continuation five compounds of the 2^{nd} generation of screening Inhibitor No. 23 (N-[5-(methanesulfonamide)-1,3,4-thiadiazol-2-yl]-6,7,8,9-tetrahydro-5H-carbazole-3-carboxamide), Inhibitor No. 29 (3-(4-chlorophenyl)sulfonyl-N-(5-isoxazol-5-yl-1,3,4-oxadiazol-2-yl)propanamide), Inhibitor No. 30 (4-[3-(5-amino-1,3,4-oxadiazol-2-yl)isoxazol-5-yl]phenol), Inhibitor No. 31 ((2R)-N-(5-isoxazol-5-yl-1,3,4-oxadiazol-2-yl)-2,3-dihydro-1,4-benzodioxine-2-carboxamide) and Inhibitor No. 32 (3-(benzenesulfonyl)-N-(5-isoxazol-5-yl-1,3,4-oxadiazol-2-yl)propanamide) were primarily tested on Caco-2, COLO 829 and MDA-MB-231 cells. Preliminary tests showed dose-dependent effects of compounds on reduced lactate generation, yet some differences of their inhibitory abilities were observed in distinct tumorigenic cell lines. However, later tests with consecutive addition of aliquots of a compound to the cells revealed high efficacy of lactate formation prevention at low 10 or 20 µM concentrations.

Based on the inhibitory effect of all selected compounds on lactate generation in tumorigenic cell lines, their effects were also tested on cell proliferation and cytotoxicity. Despite reduced lactic acid generation due to the treatment of tested tumorigenic cell lines with Inhibitors No. 3, 9, 29, 30, 31 and 32 similar cell numbers and survivals were observed in treated and un-treated tumorigenic cell lines at the end of experiments. Besides, measurements of NADH levels, glycolytic capacities and lactate excretion in treated and un-treated Jurkat cells in the presence of two compounds (Inhibitors No. 3 and 9) revealed PFK1 iso-enzymes as a target.

Because the acidification of the extracellular fluid in tumours, lactic acid is believed to be the key cause of immune escape by cancer cells. The compounds that prevent lactate excretion might be of extreme importance for the success of immunotherapies in cancer treatment. On the other hand, the selected compounds showed negligible cytostatic and cytotoxic effects on tested tumorigenic cell lines (Jurkat, Caco-2, COLO 829, MDA-MB-231) and non-tumorigenic cells (such as HEK and normal MCF 10A cells), therefore mild or no side effects on normal cells might be expected. These results suggest that inhibitors No. 3, 9, 23, 29, 30, 31, 32 significantly reduce lactate excretion by cancer cells, consequently leading to higher susceptibility of cells to the immune system, while their growth and survival is not affected. These seven compounds are thus promising inhibitors to be used in cancer therapy and treatment. Their use, either alone, either in any possible combination of inhibitors of the 1^{st} and 2^{nd} generation that inhibit different human PFK isoenzymes is the preferred embodiment of the invention, as combinations of inhibitors of the 1^{st} and 2^{nd} generation could inhibit all three types of the cancer-specific modified PFK1 enzymes. It has been observed that inhibitor efficiency decreases with time, hence the selected inhibitor or their combination is preferably administered in regular time intervals to ensure proper dosage at all times. The results shown in the examples below confirm the success of such interval treatment, as lactate formation and excretion has been successfully inhibited with low inhibitor concentrations. Described inhibitors can also be used in combination with other anticancer treatments and/or medicaments in any possible way depending on interactions between compounds. Such use may be simultaneous or sequential.

The present invention relates to the above-mentioned compounds for use as medicaments, especially for treating cancer, with the aim of drastically reducing lactate generation and secretion by the cancer cells that become more susceptible to the immune system. It is also possible to use any pharmaceutically suitable salt, solvate, structural analogue and/or derivative of inhibitors No. 3, 9, 23, 29, 30, 31 and 32 which retain the capability of docking into the ATP binding site of PFK-1 and show inhibition of overactive cancer-specific PFK enzymes. The expression derivative in this application is considered to be a compound that is derived from inhibitors No. 3, 9, 23, 29, 30, 31, and 32 by a chemical reaction. The expression derivative is also considered to cover structural analogues of the said inhibitors.

In continuation, the invention will be described in more detail based on examples and figures, which show:
- Figure 1:: Two types of ATP binding sites characteristic for a) PFK-P/PFK-M isoform, and b) PFK-L isoform, used for virtual screening of a ZINC Drugs NOW database by a supercomputer.
- Figure 2:: Lactate excretions by the Jurkat cells in the presence of selected inhibitors of the 1^{st} generation of screening followed for 48 hours. Significantly lowered lactate excretions were observed at 100 µM concentration of Inhibitor No. 3 and Inhibitor No. 9 (arrows).
- Figure 3:: Effects of selected inhibitors in the range from 10 - 50 µM on the activities of the purified recombinant native human (85 kDa) and modified (47 kDa) 6-phosphofructo-1-kinase (PFK-M) enzymes by inhibitors No. 3 and No. 9.
- Figure 4:: Lactate excretion by tumorigenic Jurkat cells in the presence of different concentrations of Inhibitor No. 3, a) and lactate excretion by the Jurkat cells in the presence of different concentrations of Inhibitor No. 9, b).
- Figure 5:: Total cell numbers of tumorigenic Jurkat cells in the presence of different concentrations of Inhibitor No. 3, a) and in the presence of different concentrations of Inhibitor No.9, b) at 72 hours.
- Figure 6:: The long-term effect of Inhibitors No. 3 and No. 9 at 100 µM concentration on tumorigenic Jurkat cells, a) lactate excretion, b) a total number of cells, c) survival.
- Figure 7:: NADH levels in Jurkat cells 24 hours after the addition of Inhibitors No. 3 and 9 at different concentrations.
- Figure 8:: Extracellular acidification rate (ECAR, panel a, c), and oxygen consumption rate (OCR, panel b, d) were determined at proliferating Jurkat cells with no (control) or with added Inhibitors No. 3 and No. 9 at 100 µM concentration by the Seahorse assay. ATP synthase inhibitor oligomycin A and mitochondrial un-coupler Carbonyl cyanide-4-(trifluoromethoxy)phenylhydrazone (FCCP) were added to the cells 20 minutes after the start of the experiment.
- Figure 9:: Effect of different concentrations of Inhibitor No. 3 on lactate excretion by non-tumorigenic human embryonic kidney cells (HEK 293) (panel a) and non-tumorigenic epithelial cells of the mammary gland (MCF 10A) (panel b).
- Figure 10:: Lactate excretions by the Colo-2 cells a), CACO 829 cells b), and MDA-MB-231 cells c), in the presence of selected inhibitors of the 2^{nd} generation of screening that were followed for 90 hours. Significantly lowered lactate excretions were observed at 100 µM concentration of some inhibitors marked by arrows.
- Figure 11:: Effects of selected inhibitors in the range from 10-50 µM on the activities of the purified recombinant native human (85 kDa) and modified (70 kDa) 6-phosphofructo-1-kinase (PFK-L) enzymes by the Inhibitors No. 23, 29, 30, 31, 32.
- Figure 12:: Lactate excretion by tumorigenic Caco-2 cells in the presence of different concentrations of Inhibitor No. 23, a) 29, b), 30 c) 32, d) and total cell numbers of Caco-2 cells, e) in the presence of different concentrations of Inhibitors at 91 hours.
- Figure 13:: Lactate excretion by tumorigenic COLO 829 cells in the presence of different concentrations of Inhibitor No. 23, a) 29, b), 30 c) 31, d) and total cell numbers of COLO 829 cells, e) in the presence of different concentrations of Inhibitors at 91 hours.
- Figure 14:: Lactate excretion by tumorigenic MDA-MB-231 cells in the presence of different concentrations of Inhibitor No. 23, a) 29, b), 30 c) 31, d) and total cell numbers of MDA-MB-231 cells, e) in the presence of different concentrations of Inhibitors at 91 hours.
- Figure 15:: Lactate excretion by tumorigenic Caco-2 cells after the sequential re-insertion of low concentrations of Inhibitors No. 3, a) 9, b) 23, c) 29, d) 30 e) at indicated time points (arrows). Total cell numbers of Caco-2 cells after the sequential re-insertion of low concentrations of inhibitors at 91 hours.
- Figure 16:: Lactate excretion by tumorigenic COLO 829 cells after the sequential re-insertion of low concentrations of Inhibitors No. 3, a) 9, b) 23, c) 29, d) 30 e) at indicated time points (arrows). Total cell numbers of COLO 829 cells after the sequential re-insertion of low concentrations of Inhibitors at 91 hours.
- Figure 17:: Lactate excretion by tumorigenic MDA-MB- 231 cells after the sequential re-insertion of low concentrations of Inhibitors No. 3, a) 9, b) 23, c) 29, d) 30 e) at indicated time points (arrows). Total cell numbers of MDA-MB-231 cells after the sequential re-insertion of low concentrations of Inhibitors at 91 hours.

In all figures, the data are representative of three independent measurements and are presented as means ± standard deviation.

### Examples

### Example 1

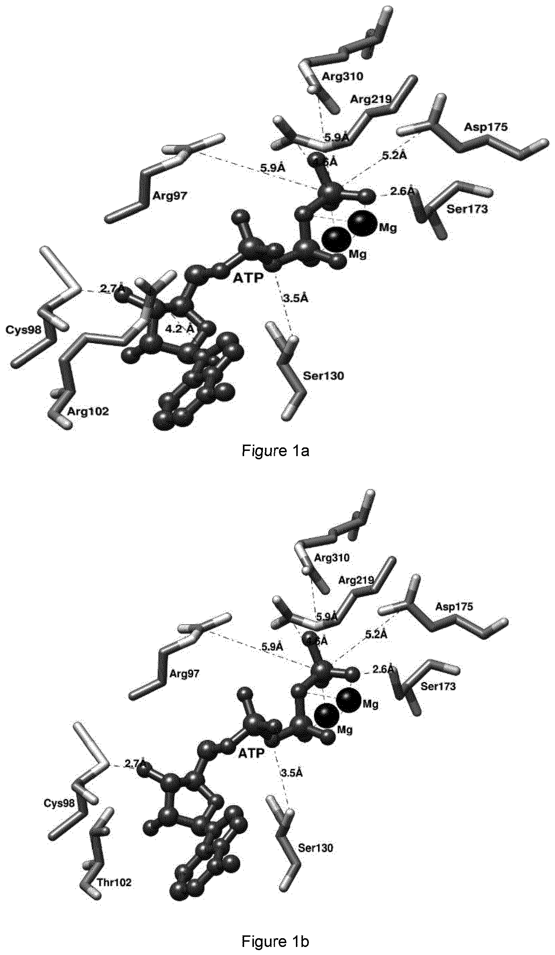

To find a drug that would inhibit overactive modified PFKs, a large-scale target based virtual screening was performed by docking small-molecule ligands into the ATP binding site of the human PFK-P (PDB 4xyj) by a supercomputer. Human cells express three types of PFK1s isoforms, namely PFK-P, PFK-M and PFK-L. Identical ATP binding sites were found in M and P isoforms (Figure 1), therefore ATP binding site of PDB4xyj crystal stricture (Figure 1a) was used for the screening of the 1^{st} generation of inhibitors. PFK-L ATP binding site differs from the former two isoforms in one amino acid. The model with modified ATP binding site with threonine at position 102 was generated by using the most probable rotamere obtained from the Dunbrack database (http://dunbrack.fccc.edu/bbdep2010/) that exhibited no steric clashes. The modified model was used to virtually screen the ZINC Drugs NOW database by the supercomputer (2^{nd} generation of inhibitors). In Figure 1a the amino acid residues of the PFK-P ATP binding site with the native ligand ATP is showen. In Figure 1b, PFK-L characteristic modified model with substituted arginine with threonine and ATP molecule is presented. Distances among specified moieties on amino acid residues and ATP molecule are shown corresponding to the interactions between the protein and ATP molecule on both figures.

### Example 2

All purchased compounds were supplied as 5mg lyophilized powder and were dissolved in DMSO to the final concentration of 10 mM.

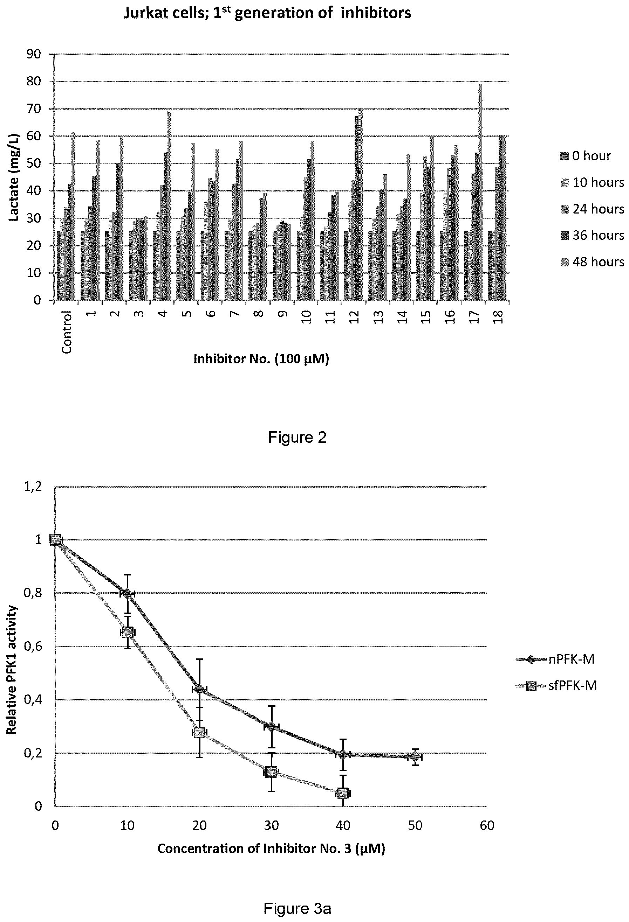

Initially, all the compounds of the 1^{st} generation of screening were preliminary tested for the prevention of lactate generation by acute lymphoblastic leukemia Jurkat cells that were grown in the RPMI 1640 GlutaMAX medium (Thermo Fisher) supplemented with 10% of fetal bovine serum (FBS). The cells were incubated in 1 mL volume (24 well plates) at 5% CO₂ and 37°C. The inhibitors were added in 100 µM concentration. As a control, equivalent amount of DMSO as used for the addition of inhibitors was supplemented to the medium. The tests started with 1×10⁵ cells per mL. Extracellular lactate excreted by the Jurkat cells was measured in the supernatant of the growth medium after the cells were removed by centrifugation at 1500 rpm for 5 minutes. Lactate concentration was determined enzymatically using L-Lactatic acid kit (K-LATE, Megazyme, Ireland). The tests of lactate excretion showed that inhibitors No. 3 and No. 9 effectively prevented excretion (arrows). Less efficient reduction of lactate overflow in respect to the control was observed also by the compounds No. 8, 11, 13 and 14 (Figure 2).

### Example 3

Selected compounds were tested for in vitro inhibition of isolated recombinant human native and modified PFK-M enzymes. The alignment of amino acid residue sequences of all three human PFK1 isoforms (PFK-P platelet type; PFK-M muscle type; PFK-L liver type) by CLUSTALW revealed identical amino acid residues of the ATP binding site in the PFK-P and PFK-M isoform.

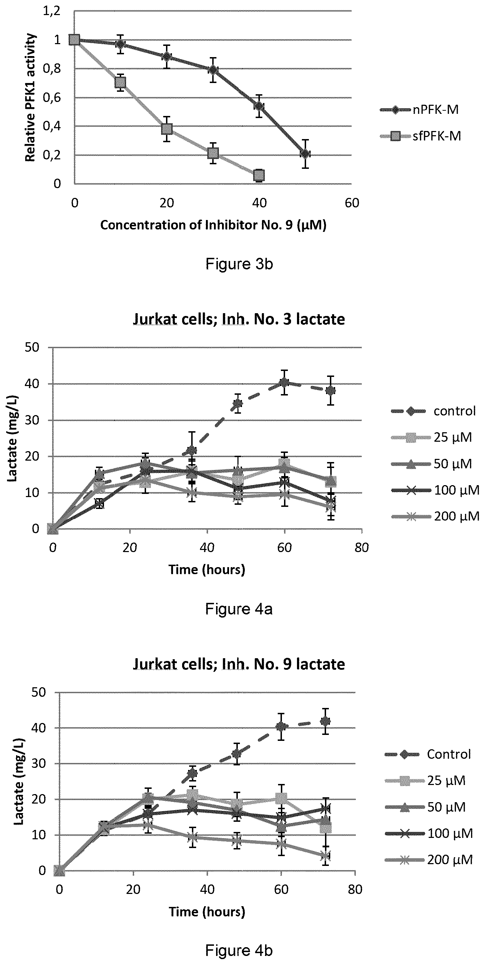

Recombinant native muscle type PFK-M of 780 amino acid residues (mass 85,183 Da) and short, cancer specific fragments of 443 residues (mass 47,835 Da) were synthesized after the insertion of the full length human and truncated cDNA (Clone ID2964710) into *pfk1, pfk2* null yeast *S*. *cerevisiae* derivative HD 114-8D as described previously (Smerc et al., 2011, doi:10.1371/journal.pone.0019645; Andrejc et al., 2017, doi:10.1186/s12896-017-0362-5). The yeast transformant encoding the native human PFK-M enzyme was designated as n*PFKM* strain while transformant encoding cancer specific shorter fragments was designated as sf*PFKM* strain. Recombinant enzymes were purified to homogeneity according to the protocol published previously (Smerc et al., 2011). Because the shorter fragments proved to be extremely unstable under diluted conditions (Smerc et al., 2011), all PFK1 activities were measured in the buffer with 20% of polyethylene glycol (PEG) as recommended previously (Reinhart et al. http://www.ncbi.nlm.nih.gov/pubmed/6448853). The inhibition of PFK1 activities were recorded spectrophotometrically (Lambda 25, Perkin-Elmer, Boston. US), and the enzyme kinetics measured as described previously (Smerc et al., 2011). For the enzymatic measurements, all the substrates (F6P and ATP), auxiliary enzymes, NADH and inhibitors dissolved in DMSO were added first to the buffer with PEG. After thorough mixing of the buffer with all ingredients, the enzymatic reaction started by the insertion of the purified recombinant enzymes. Out of eighteen tested compounds of the 1^{st} generation of screening, two Inhibitors (No. 3 and No. 9) showed strong inhibition of highly active sfPFK-M fragments, with half maximal inhibitory concentrations (IC₅₀ values) of approximately 15 and 17 µM respectively. Although Inhibitor No.3 remarkably reduced also the activity of native nPFK-M enzyme (Figure 3a), not so strong inhibition of this enzyme was recorded by the Inhibitor No.9 (Figure 3b).

Because no in vitro PFK-M inhibition could be detected by the compounds No. 8, 11, 13 and 14, they might reduce lactate production by another unspecified mechanism. In continuation only Inhibitor No. 3 and Inhibitor No. 9 were further tested.

### Example 4

The main goal for a search of appropriate cancer specific PFK-M inhibitors was to partially decrease PFK1 activities in the cancer cells to the levels found in the normal cells.

Consequently, deregulated glycolytic flux should be reduced and NADH formation diminished. Reduced rate of cytosolic NADH formation should prevent the necessity of redundant NADH re-oxidation by lactate dehydrogenase and therefore prevent lactate generation and excretion. It should be stressed that apart from reducing lactate generation it was very important to find inhibitors with minimal side effects. In this regard, selected compounds (Inh. No. 3 and No. 9) were tested also for cytostatic and cytotoxicity effects.

Measurements of lactate excretion in the tumorigenic Jurkat cells and the cells treated with two selected inhibitors at different concentrations were repeated by more detailed measurements. The growth conditions were identical to those described in Experiment 2. No significant difference in lactate excretion could be observed by the treated and untreated cells during the initial stage of growth that lasted for approximately 12 hours. Later on lactate excretion by the Jurkat cells ceased in the presence of both inhibitors. The most effective and rapid prevention of lactate accumulation was observed at 200 µM concentration of both inhibitors while inhibitors at lover concentrations averted lactate production later, at about 24 hours with Inhibitor No. 3 (Figure 4a) and at 36 hours with Inhibitor No. 9 (Figure 4b).

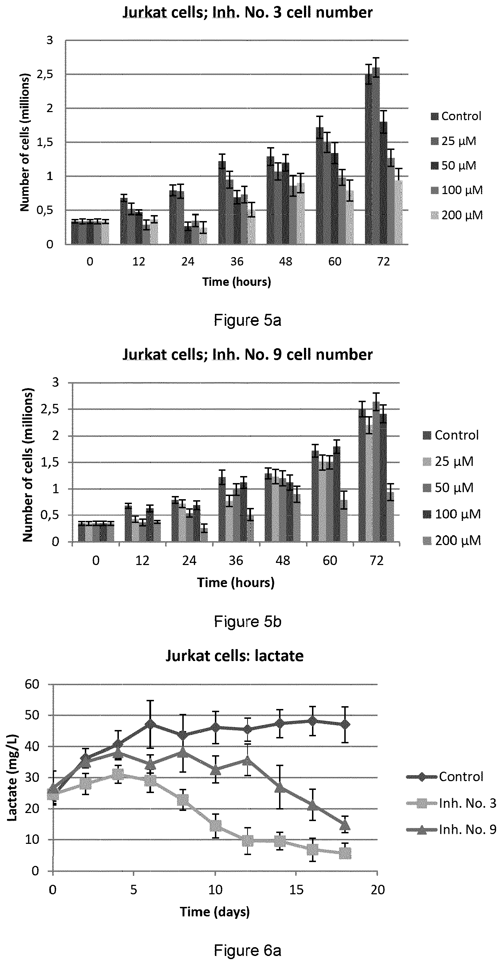

The effects of both inhibitors were tested also on growth of the non-adherent Jurkat cells. Total amount of cells and the number of dead cells was determined by the Countess (Invitrogen, Carlsbad, CA). While Inhibitor No. 3 caused remarkable reduction of growth rate at concentrations higher than 25 µM (Figure 5a), no effect of Inhibitor No. 9 was observed on growth at higher concentrations (Figure 5b). Mostly no significant negative effects on the survival of the Jurkat cells were observed by Inhibitor No. 3 (3,21±0,27% dead cells) and Inhibitor No. 9 (3,76±0,3 % dead cells) after the cells were stained with Tripan blue.

### Example 5

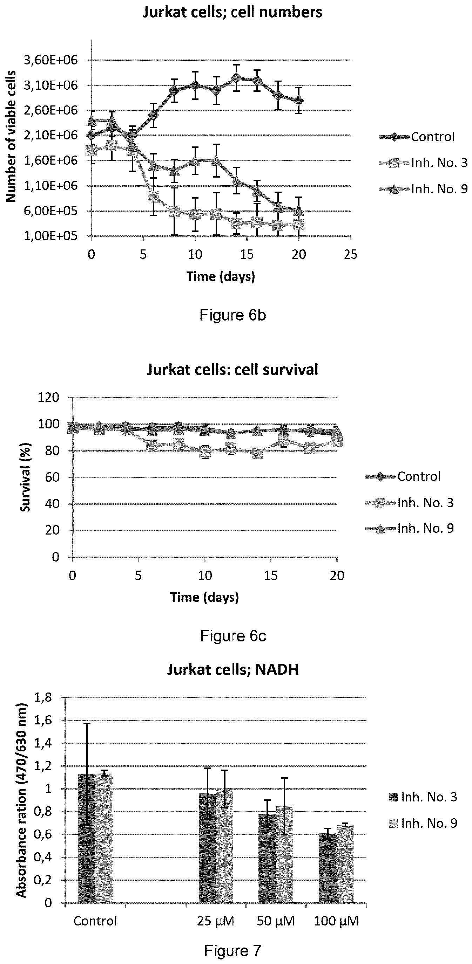

To check, whether the inhibition is stable or any mechanisms of drug resistance are triggered, the Jurkat cells were grown by fed-batch cultivation for 18 days using the growth conditions as described previously (Example 2) with some modifications. The cells were grown in 24 well plates and the incubation started with 250 µL of medium without or with Inhibitor No. 3 (100 µM) or Inhibitor No. 9 (100 µM) with starting number of approximately 5×10⁵ cells per mL. After 48 hours, the samples for determination of cell numbers, survival and extracellular lactate concentration were taken. Immediately after sampling, the total volumes of the cultures were doubled by adding 250 µL of fresh medium without or with inhibitors. After next 48 hours another set of samples was taken and the culture medium volume doubled again by the addition of the fresh medium (500 µL) without or with inhibitors. When the cells grew for additional 48 hours in a total volume of 1 mL, samples were taken again, then 250 µL aliquots of culture were transferred to the new wells and the volumes immediately doubled with the fresh medium without or with inhibitors. The volumes of the culture medium were continuously doubled by the fresh medium without or with inhibitors at 48 hours intervals for 18 days. In the experiment no major changes in cell numbers, lactate excretion and cell viability could be observed with or without inhibitors during the first 48 hours. Later, the cell numbers started to increase in the medium with no inhibitors while they started to decline in the presence of both inhibitors. A lag phase of about 48 to 96 hours was detected also in lactate production and survival where no significant changes among all three samples could be detected. However, later the number of cells steadily declined in the presence of Inhibitor No. 9 and even more rapidly with Inhibitor No. 3. Viability of the Jurkat cells remained unchanged in the culture with untreated cells and cells with Inhibitor No. 9 while slight decrease of viability was observed only in a medium with added Inhibitor No. 3 (Figure 6 a, b, c). These data suggest slower growth of the cells with inhibitors after 5 days of incubation but the reduced cell number seems merely to be a consequence of slower growth and not reduced survival (the generation time of the treated cells seems to be longer than 48 hours). Similarly, to the data in Figure 4a and b, also in the long term experiment, slow reduction of lactate generation rate was observed after the addition of Inhibitors No. 3 and No. 9 that lasted for about 24-48 hours. The tests shown in the example 5 confirm stabile inhibitory activities of both compounds in the tumorigenic Jurkat cells. Apparently, no mechanisms were triggered that could cause drug resistance during the prolonged cultivation.

### Example 6

By inhibiting modified PFK-M enzyme in the cancer cells, glycolytic flux should be decreased and concomitantly NADH levels reduced. To verify that, the amounts of reduced pyridine nucleotide was measured by NADH/NAD fluorometric assay kit (Abcam). The Jurkat cells were seeded into the wells of 96 wells plate at 5×10⁵ cells per mL. The untreated cells (control) and the cells with specified amounts of added Inhibitors No. 3 and No. 9 were incubated for 24 hours. The results revealed slightly higher NADH levels in the untreated Jurkat cells while the NADH levels steadily decreased in dose dependent manner in the tumorigenic cells with Inhibitors No. 3 or No. 9 (Figure 7). To some extent stronger decrease of the amount of reduced nucleotide was observed in the presence of Inhibitor No. 3.

### Example 7

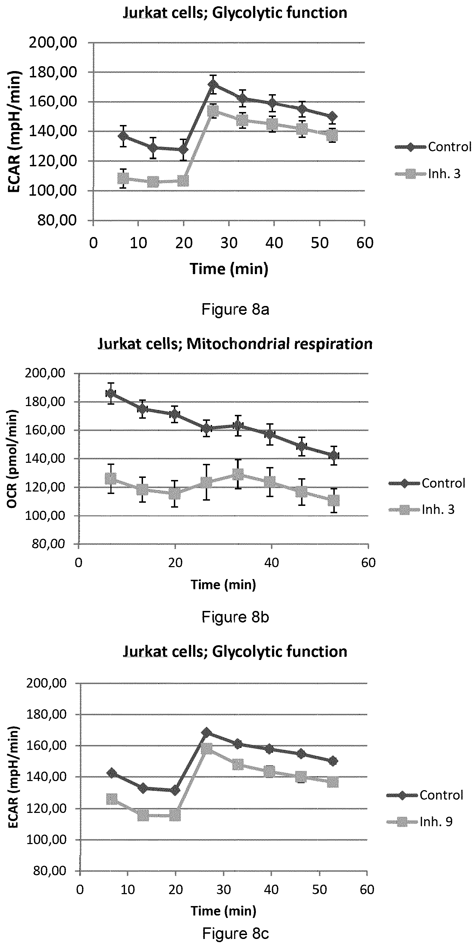

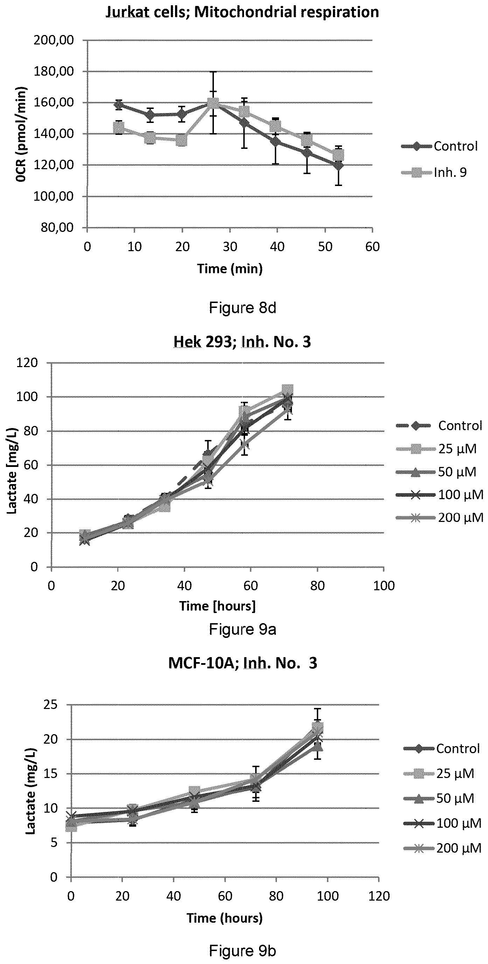

The efficiency of Inhibitors No. 3 and No. 9 was also tested by the determinations of glycolytic capacities in Jurkat cells in the presence and absence of inhibitors. For this purpose, Seahorse XF assay (Agilent Technology) was performed that enables non-destructive measurements of extracellular acidification rate (ECAR) and oxygen consumption rate (OCR) of tested cells. The cells were incubated in RPMI 1650 GlutaMAX medium with 10% FBS and 24 hours before the experiment inhibitors were added (100 µM). Non-adherent Jurkat cells (1×10⁶ per mL) were stuck to the bottom of the wells by poly-I-lysine. The experiment was conducted essentially as recommended by Seahorse XF protocol and after 20 minutes of incubation, 1 µM of oligomycin A (ATP synthase inhibitor) and 0,25 µM of Carbonyl cyanide-4-**(trifluoromethoxy)phenylhydrazone** (FCCP) (mitochondrial oxidative phosphorylation uncoupler) were added to the cells. As shown in Figure 8, untreated Jurkat cells revealed higher ECAR and OCR values in respect to the cells treated with Inhibitor No. 3 (Figure 8a, b) or Inhibitor No. 9 (Figure 8ac, d) prior to the addition of oligomycin A and FCCP. Reduced lactate excretion by the cells treated with both inhibitors might be responsible for reduced ECARs. Although the addition of oligomycin A and FCCP increased the extracellular acidification rate by the cells treated with both inhibitors, similar increase of ECAR was also observed in the untreated cells. However, inhibition of ATP synthase and uncoupling oxidative phosphorylation, phenomena that both affect mitochondrial ATP formation and respiration, caused more intense increase of oxygen consumption rate by the cells treated with both inhibitors in respect to the untreated cells, yet slightly stronger affect was observed with Inhibitor 9. It implies that inhibited PFK1 enzymes might reduce glycolytic flux and prevent the need for pyruvate reduction into lactate by re-oxidizing redundant cytosolic NADH. Under such conditions pyruvate can enter mitochondria, replenishing tri-carboxylic acids (TCA) cycle and fuelling oxidative respiration. Reduced sensitivity of untreated tumorigenic cells to oligomycin A reveals that glycolytic ATP formation prevails over mitochondrial respiration.

### Example 8

The effect of Inhibitor No. 3 on lactate excretion was tested also on two non-tumorigenic cell lines. First, fast proliferating human embryonic kidney (HEK 293) cells were assessed for lactate formation in the presence of Inhibitor No. 3. RPMI 1640 GlutaMAX medium (Thermo Fisher) supplemented with 10% FBS was used as a growth medium. During the 72 hours of growth in a medium with different concentrations of inhibitor, no significant reduction of lactate generation was observed regardless of the amount of inhibitor in the medium. About 95 mg/L of lactate were detected after 72 hours of incubation by untreated cells (control) and by the cells growing in the presence of Inhibitor No. 3 (Figure 9a). Similar results were recorded also by non-tumorigenic MCF-10A cell line isolated form the epithelial cells of the mammary gland. The cells were cultivated in MEBM medium supplemented by additives that were obtained as a MEGM Kit from Lonza/Clonetics Corporation. Characteristically these cells grew slower than HEK 293 cells with correspondingly slower lactate excretion rate. After 96 hours of incubation, only about 27 mg/L of lactate was detected in the medium. Again, no influence of different concentrations of Inhibitor No. 3 on lactate generation could be observed (Figure 9b).

### Example 9

Threonine residue instead of arginine was found at the position 102 of the ATP binding site of the PFK-L isoform as described in the example 1. Therefore, 2^{nd} generation of screening was conducted to select the compounds that would preferentially dock to the human PFK-L specific ATP binding site. Virtual screening of ZINC Drugs NOW database revealed 15 new compounds with possible inhibitory function that were purchased from Enamine or Chembridge.

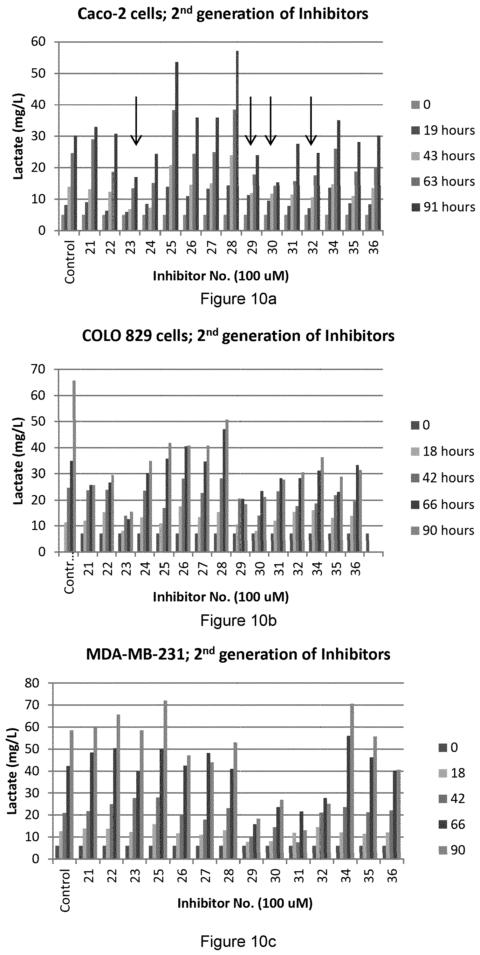

Preliminary in vivo tests were run by measuring lactate production by three different tumorigenic cell lines. Again, 100 µM concentrations of tested compounds have been added to the medium. For the experiment, the following tumorigenic cells lines originating from different tumours were tested: Caco-2 cells (Figure 10a), originating from colorectal adenoma carcinoma; COLO 829 cells (Figure 10b), isolated from melanoma; and MDA-MB-231 cells (Figure 10c) derived from metastatic phase of mammary/breast cancer. All adherent cell lines were grown in the RPMI 1640 GlutaMAX substrate (Thermo Fisher) supplemented with 10% FBS. Although, specific compounds showed different effectiveness of lactate generation inhibition in individual tumorigenic cell lines (marked by arrows), Inhibitors No. 23, 29, 30, 31 and 32 were selected for more detailed assessment.

### Example 10

For the in vitro tests, inhibitory characteristics of selected compounds on the activities of purified native and modified PFK-L enzymes were determined. Recombinant human native liver type PFK-L of 780 amino acid residues (mass 85,018 Da) and recombinant shorter PFK-L fragment of 645 residues (mass 69.942 kDa) were synthesized in HD 114-8D yeast strains designated as *nPFKL* and *sfPFKL.* Both inserted genes were prepared by Gibson assembly cloning using P17858-1 (https://www.uniprot.org) gene nucleotide sequence as a template.

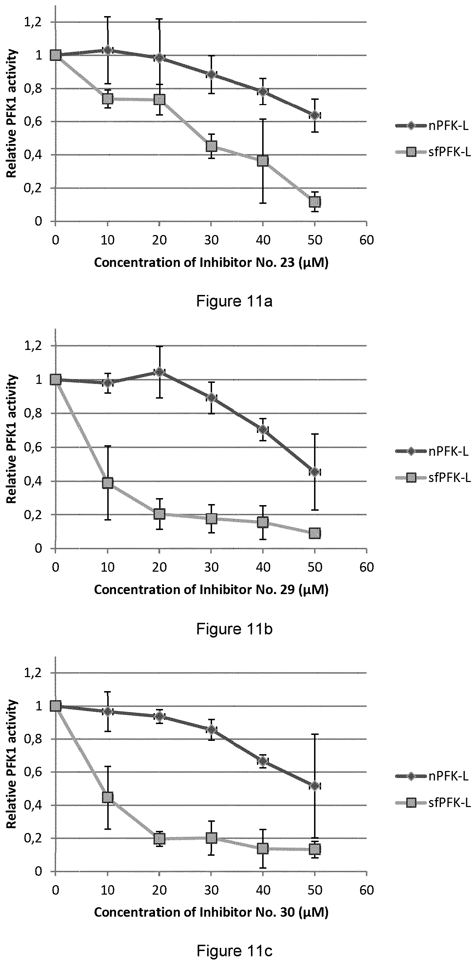

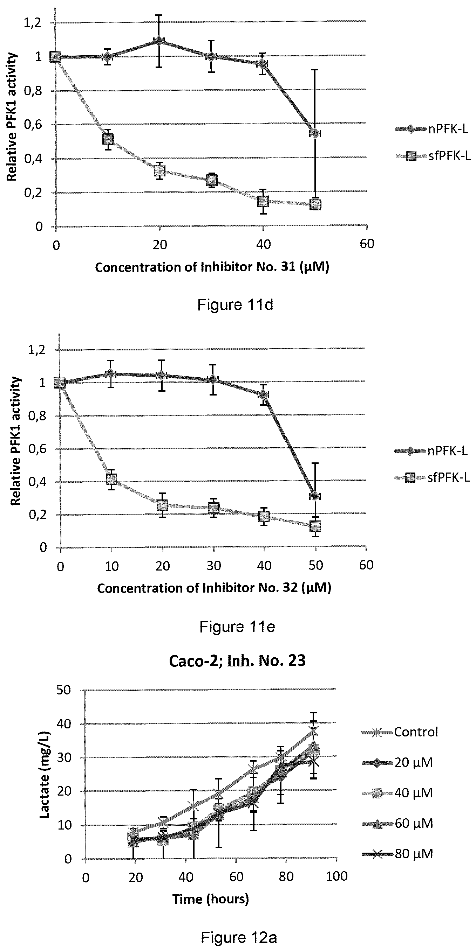

Inhibitors No. 29 and 32 showed strong inhibition of isolated recombinant sfPFK-L enzyme (IC₅₀ = 8 µM each) (Figure 11b and 11c). Similar half maximal inhibitory concentration was observed also for Inhibitors No. 30 and 31 (IC₅₀ = 10 µM each) (Figure 11d and 11e). The weakest inhibition was recorded with Inhibitor No. 23 (IC₅₀ = 27 µM)(Figure 11a). All tested inhibitors of the 2^{nd} generation showed weaker inhibition of the nPFK-L isoform (Figure 11a, b, c, d, e).

### Example 11

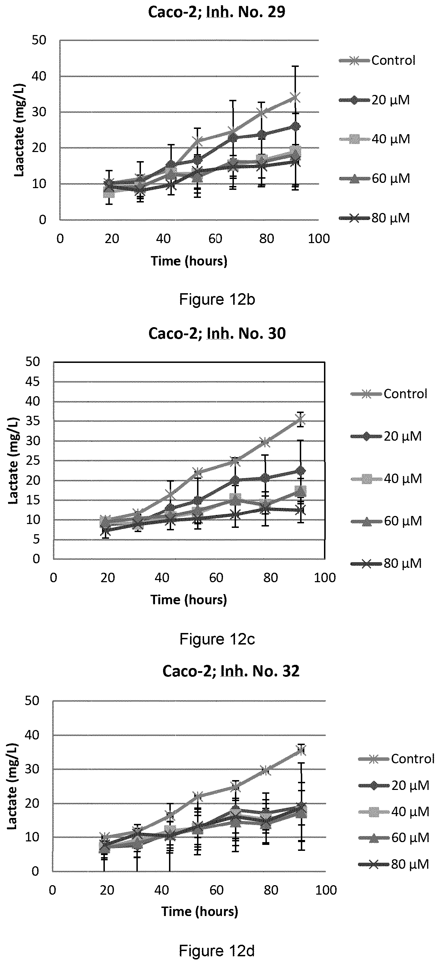

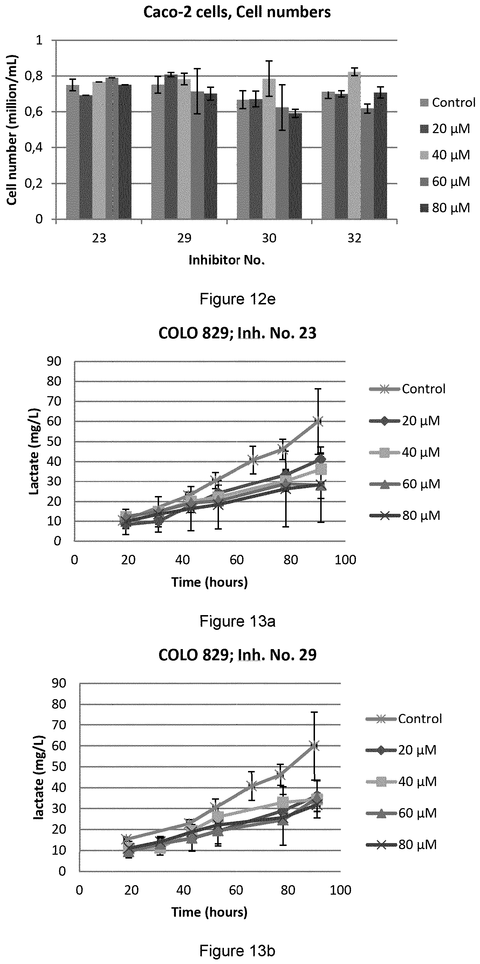

Lactate generation, growth yield and survival under normal and inhibitory conditions of three different tumorigenic cells lines were determined in more detailed measurements. On the basis of preliminary results, the Caco-2 cells were tested for Inhibitors No. 23, 29, 30 and 32. Inhibitor No. 23 showed strong inhibition of lactate generation during the first 36 hours of inhibition in concentration range from 20-80 µM, however later the inhibitory effect was lost (Figure 12a). Inhibitors No. 29 (Figure 12b) and 30 (Figure 12c) effectively reduced the lactate accumulation in the medium in a dose dependent manner, while Inhibitor No. 32 (Figure 12d) exhibited strong inhibition at all tested concentrations. By determination of total cell numbers at the end of incubation (92 hours), not much difference among control and cells grown in the presence of inhibitors was detected, however with Inhibitors No. 29 and 30 minor dose dependent reductions in total cell numbers were observed (Figure 12e). Cytotoxicity was measured by the Lactate dehydrogenase (LDH) kit (Roche). No cytotoxic effects of inhibitors were observed. The average percent of dead cells in the control and in the presence of different concentrations of inhibitors were as follows: Inh. 23 (3,35±0,08%), Inh. 29 (4,95±0,27%), Inh. 30 (4,32±0,51%), Inh. 32 (3,97±0,18%). Additionally, the following proportions of dead cells in the presence of inh. 3 (3,99±0,08%) and 9 (3,33±0,14%) of Caco-2 cells were also determined. No dose dependent decrease of survival was observed by any inhibitor.

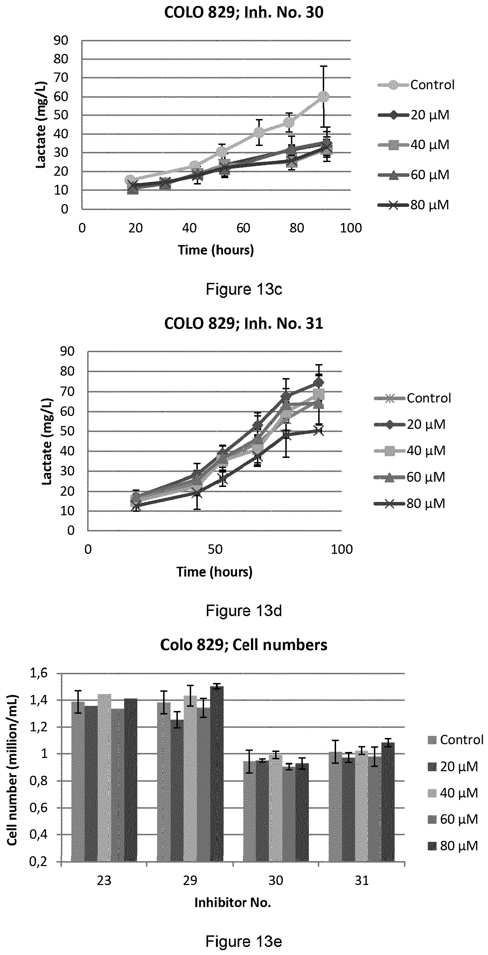

On the basis of preliminary results, inhibitors No. 23, 29, 30 and 31 were tested on COLO 829 cells. The strongest reductions of lactate excretion were observed with Inhibitors No. 29 (Figure 13b) and No. 30 (Figure 13c) where strong reduction of acid production was detected even at low concentrations. Slightly weaker inhibition was found by inhibitor No. 23 (Figure 13a) where some dose dependent reduction of lactate excretion was observed. Inhibitor No. 31 showed some inhibition, however lower inhibitor concentrations showed just minor decreases of lactate generation (Figure 13d). No significant discrepancies of total cell numbers in respect to the control were observed due to the different concentrations of inhibitors in the medium (Figure 13e). The average percent of dead COLO 829 cells in the control and in the presence of different concentrations of inhibitors were as follows: inh. 23 (9,23±0,6%), inh. 29 (9,31±0,73%), inh. 30 (8,27±0,49%), inh. 31 (8,26±0,38%). Additionally, cytotoxicity of inh. 3 (5,01±1,08%) and 9 (3,65±0,78%) for COLO 829 cells were also determined. No dose dependent decrease of survival was observed by any inhibitor.

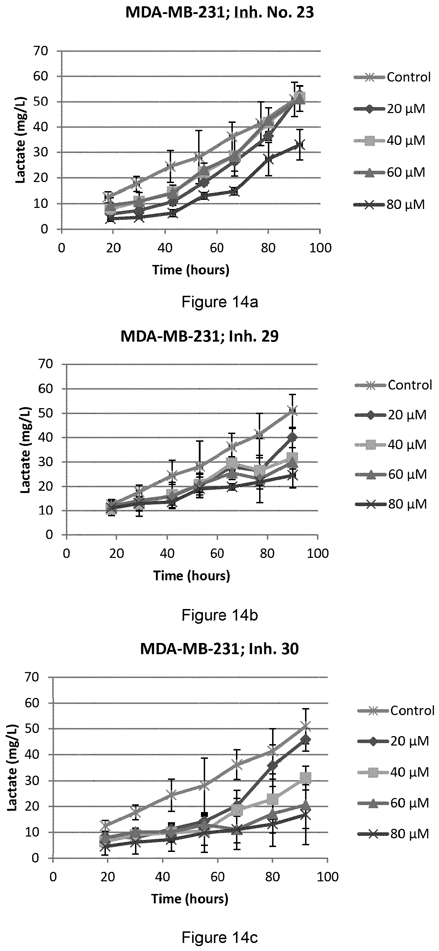

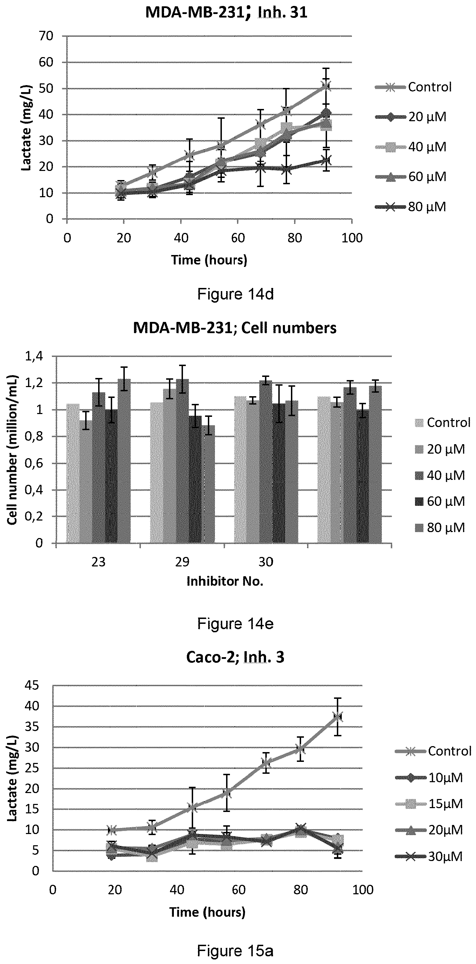

Inhibitors No. 23, 29, 30 and 31 were tested for preventing lactate generation by the MDA-MB-231 cells. Similar dose dependent inhibition of lactate excretion was observed by inhibitors No. 29 (Figure 14b) and No. 31 (Figure 14d). Inhibitors No. 23 (Figure 14a) and No. 30 (Figure 14c) showed strong inhibition during the first 42 hours (Inh. No. 23) and 54 hours (Inh. No. 30) while later on lactate generation re-appeared. No significant discrepancies of total cell numbers were observed (Figure 14e) due to the different concentrations of inhibitors in the medium. However, dose dependent decrease of cell numbers was observed with Inhibitor No. 29, similarly as it was found with Caco-2 cells (Figure 12e). The average percent of dead MDA-MB-231 cells in the control and in the presence of different concentrations of inhibitors were as follows: inh. 23 (2,98±0,2%), inh. 29 (3,37±0,39%), inh. 30 (3,4±0,21%), inh. 31 (6,87±0,44%). Additionally, cytotoxicity of inh. 3 (7,2±0,47%) and 9 (6,59±0,29%) for MDA-MB-231 cells were also determined. No dose dependent decrease of survival was observed by any inhibitor.

### Example 12

A decrease in inhibitory effectiveness of some compounds was observed after certain period of time (Example 11). Inhibitory strength seemed to disappear sooner at lower concentrations, suggesting degradation of the compound in the medium or in the cells. The most obvious examples of such reductions were observed with Inhibitor No. 23 in Caco-2 and MDA-MB-231 cells (Figure 12a and Figure 14a respectively). Therefore, the influence of selected inhibitors was assessed by sequential re-insertion of low concentrations of inhibitors at indicated time points. The inhibitors were first added to the medium 8 hours after the inoculation followed by periodically additions at 24-hour intervals (marked by arrows).

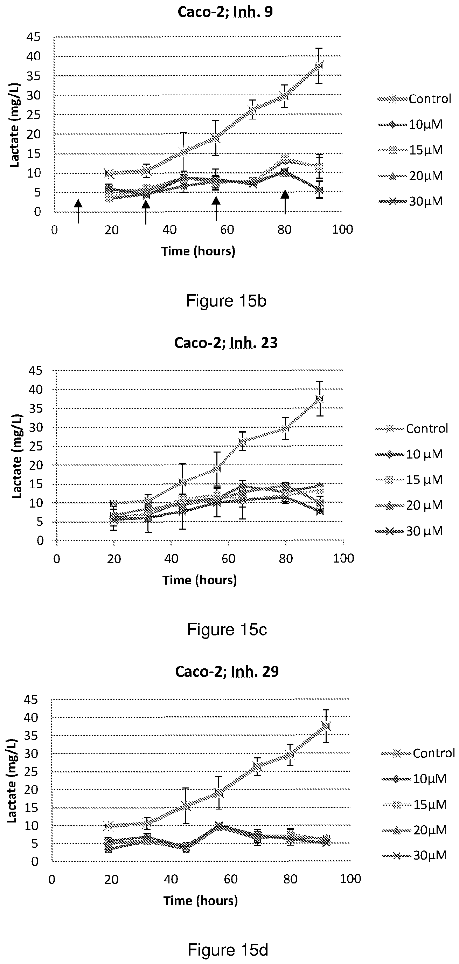

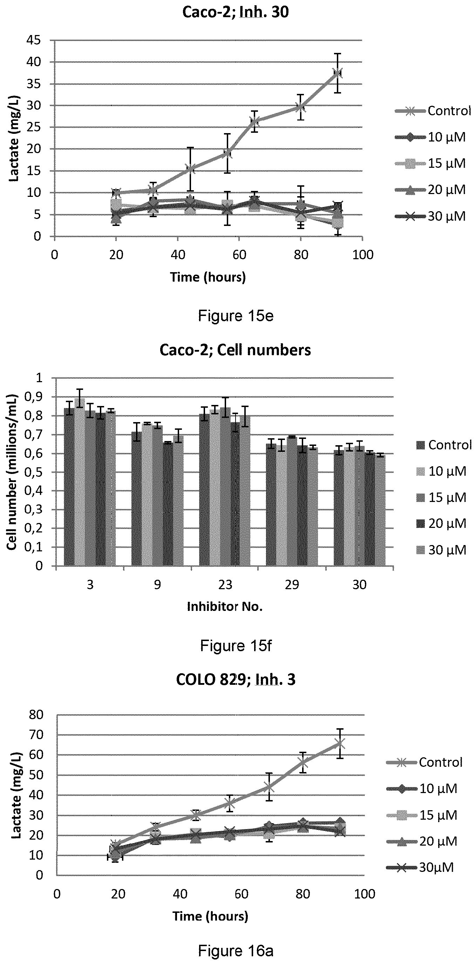

In Caco-2 cells strong inhibition of lactate formation was observed with all tested inhibitors. Almost no dose dependent effect of inhibitors could be observed at tested concentrations, implying strong inhibitions of lactate generation at the lowest 10 µM concentration. Both type of inhibitors, those selected by the 1^{st} or the 2^{nd} generation of screening, showed similar efficacy (Figures 15a, b, c, d, e). No significant cytostatic (Figure 15f) or cytotoxic effects of inhibitors could be detected after 92 hours of incubation despite of sequential addition of various concentrations of inhibitors. The following average percentages of dead cells were perceived in the medium without and media with inh. 3 (5,38±0,4%) inh. 9 (5,05±0,62%), inh. 23 (4,05±0,54%), inh. 29 (4,57±0,04%) and inh. 30 (4,68±0,28%). No detrimental effects of increased inhibitors concentrations on cell deaths have been observed.

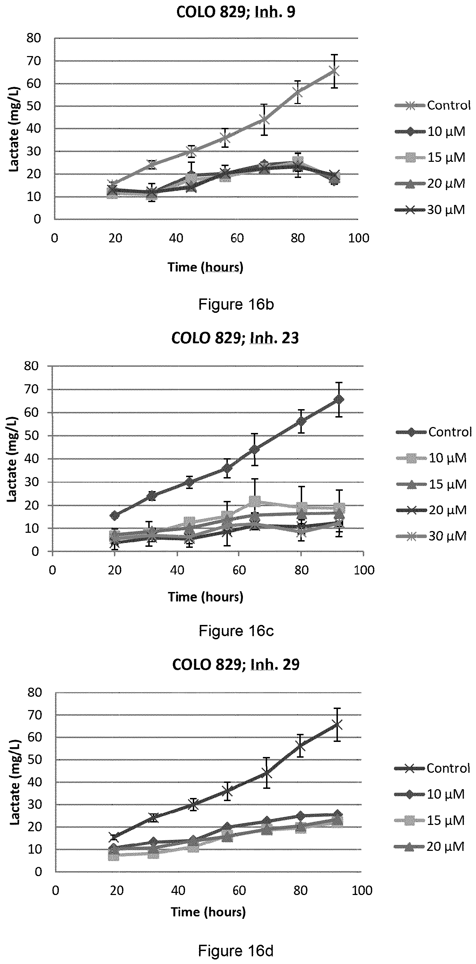

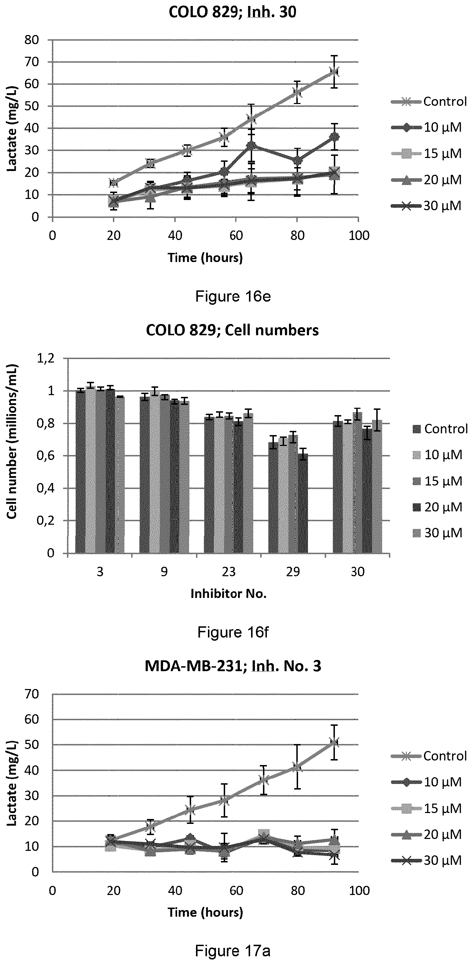

In COLO 829 cells strong reduction of lactate formation was observed at low concentrations of Inhibitors No. 3 and 9 (Figures 16a and b) after the sequential re-injection while compounds selected by the 2^{nd} generation of screening showed some dose dependency. With Inhibitors No. 29 and 30 (Figures 16d and e) slightly weaker inhibitions were observed at 10 µM concentration while with Inhibitor 23 comprehensive inhibition was detected only at 20 µM concentration or higher (Figure 16c). No significant cytostatic (Figure 16f) of cytotoxic effect of inhibitors could be detected after 92 hours of incubation despite of sequential addition of various concentrations of inhibitors. The following average percentages of dead cells were perceived in the medium without and media with inh. 3 (1,95±0,15%) inh. 9 (1,70±0,14%), inh. 23 (2,40±0,09%), inh. 29 (1,66±0,25%) and inh. 30 (1,38±0,18%). No detrimental effects of increased inhibitors concentrations on cell deaths have been observed.

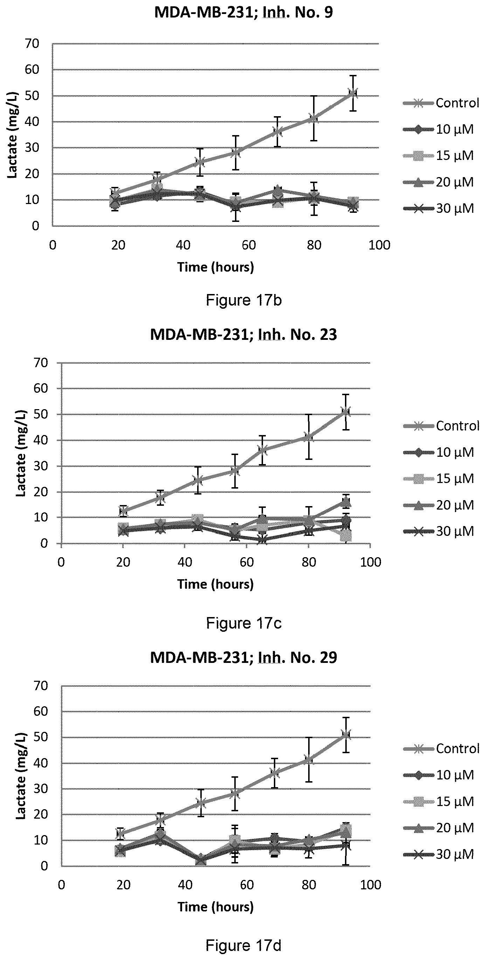

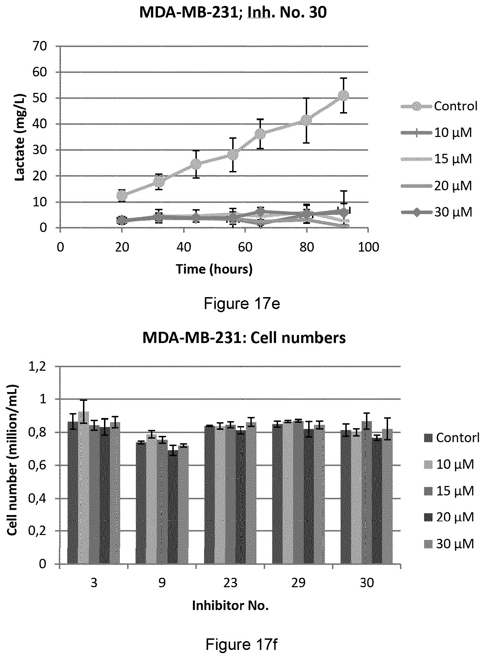

In MDA-MB-231 calls similar results to those found in Caco-2 cells were observed. Both types of compounds inhibited lactate generation very efficiently at low concentrations, if inhibitors were added periodically every 24 hours (Figures 17a, b, c, d, e). No significant cytostatic (Figure 17f) or cytotoxic effect of inhibitors could be detected after 92 hours of incubation despite of sequential addition of various concentrations of inhibitors. The following average percentages of dead cells were perceived in the medium without and with inh. 3 (2,05±0,18%), inh. 9 (1,85±0,05%), inh. 23 (2,20±0,15%), inh. 29 (1,54±0,16%) and inh. 30 (2,00±0,18%). No detrimental effects of increased inhibitors concentrations on cell deaths have been observed.

The results obtained in the above-described examples suggest that
- Inhibitor No. 3 (2-[(5-benzo[1,3]dioxol-5-yl-1,3,4-oxadiazol-2-yl)sulfanyl]-N-isoxazol-3-yl-acetamide),
- Inhibitor No. 9 (3-methoxy-6-(3-{1-[(5-methyl-1,2,4-oxadiazol-3-yl)methyl]-1H-pyrazol-3-yl}phenyl)pyridazine),
- Inhibitor No. 23 N-[5-(methanesulfonamido)-1,3,4-thiadiazol-2-yl]-6,7,8,9-tetrahydro-5H-carbazole-3-carboxamide),
- Inhibitor No. 29 (3-(4-chlorophenyl)sulfonyl-N-(5-isoxazol-5-yl-1,3,4-oxadiazol-2-yl)propanamide,
- Inhibitor No. 30 (4-[3-(5-amino-1,3,4-oxadiazol-2-yl)isoxazol-5-yl]phenol),
- Inhibitor No. 31 ((2R)-N-(5-isoxazol-5-yl-1,3,4-oxadiazol-2-yl)-2,3-dihydro-1,4-benzodioxine-2-carboxamide) and
- Inhibitor No. 32 (3-(benzenesulfonyl)-N-(5-isoxazol-5-yl-1,3,4-oxadiazol-2-yl)propanamide)
are promising compounds to be used as medicaments, particularly in cancer treatment, as they effectively lower lactate production by cancer cells while at the same time their effect on other, non-tumorigenic cells is negligible. According to the performed tests, compounds either alone either in any combination, such as inhibitors 3+9, inhibitors 3+23, inhibitors 3+29, inhibitors 3+30, inhibitors 3+31, inhibitors 3+32, inhibitors 9+23, inhibitors 9+29, inhibitors 9+30, inhibitors 9+31, inhibitors 9+32, inhibitors 23+29, inhibitors 23+30, inhibitors 23+31, inhibitors 23+32, inhibitors 29+30, inhibitors 29+31, inhibitors 29+32, inhibitors 30+31, inhibitors 30+32, inhibitors 31+32, should be used in concentrations of 200 µM or less, preferably from 5 to 100 µM, because lactate formation has been effectively reduced and no impact on cell survival has been observed. The said inhibitors, either alone or in any combination, are preferably administered in regular time intervals, such as every 10 to 48 hours, preferably every 16 to 24 hours.

## Claims

1. A compound selected in the group consisting of
- (2-[(5-benzo[1,3]dioxol-5-yl-1,3,4-oxadiazol-2-yl)sulfanyl]-N-isoxazol-3-yl-acetamide),
- (3-methoxy-6-(3-{1-[(5-methyl-1,2,4-oxadiazol-3-yl)methyl]-1H-pyrazol-3-yl}phenyl) pyridazine),
- N-[5-(methanesulfonamido)-1,3,4-thiadiazol-2-yl]-6,7,8,9-tetrahydro-5H-carbazole-3-carboxamide),
- (3-(4-chlorophenyl)sulfonyl-N-(5-isoxazol-5-yl-1,3,4-oxadiazol-2-yl)propanamide,
- (4-[3-(5-amino-1,3,4-oxadiazol-2-yl)isoxazol-5-yl]phenol),
- ((2R)-N-(5-isoxazol-5-yl-1,3,4-oxadiazol-2-yl)-2,3-dihydro-1,4-benzodioxine-2-carboxamide) and
- (3-(benzenesulfonyl)-N-(5-isoxazol-5-yl-1,3,4-oxadiazol-2-yl)propanamide)
or their pharmaceutically acceptable salts or solvates, for use as a medicament.

2. Compound (2-[(5-benzo[1,3]dioxol-5-yl-1,3,4-oxadiazol-2-yl)sulfanyl]-N-isoxazol-3-yl-acetamide), compound (3-methoxy-6-(3-{1-[(5-methyl-1,2,4-oxadiazol-3-yl)methyl]-1H-pyrazol-3-yl}phenyl) pyridazine), compound N-[5-(methanesulfonamido)-1,3,4-thiadiazol-2-yl]-6,7,8,9-tetrahydro-5H-carbazole-3-carboxamide), compound (3-(4-chlorophenyl)sulfonyl-N-(5-isoxazol-5-yl-1,3,4-oxadiazol-2-yl)propanamide, compound (4-[3-(5-amino-1,3,4-oxadiazol-2-yl)isoxazol-5-yl]phenol), compound ((2R)-N-(5-isoxazol-5-yl-1,3,4-oxadiazol-2-yl)-2,3-dihydro-1,4-benzodioxine-2-carboxamide) or compound (3-(benzenesulfonyl)-N-(5-isoxazol-5-yl-1,3,4-oxadiazol-2-yl)propanamide) or their pharmaceutically acceptable salts or solvates for use according to claim 1 in cancer therapy.

3. Compound (2-[(5-benzo[1,3]dioxol-5-yl-1,3,4-oxadiazol-2-yl)sulfanyl]-N-isoxazol-3-yl-acetamide), compound (3-methoxy-6-(3-{1-[(5-methyl-1,2,4-oxadiazol-3-yl)methyl]-1H-pyrazol-3-yl}phenyl) pyridazine), compound N-[5-(methanesulfonamido)-1,3,4-thiadiazol-2-yl]-6,7,8,9-tetrahydro-5H-carbazole-3-carboxamide), compound (3-(4-chlorophenyl)sulfonyl-N-(5-isoxazol-5-yl-1,3,4-oxadiazol-2-yl)propanamide, compound (4-[3-(5-amino-1,3,4-oxadiazol-2-yl)isoxazol-5-yl]phenol), compound ((2R)-N-(5-isoxazol-5-yl-1,3,4-oxadiazol-2-yl)-2,3-dihydro-1,4-benzodioxine-2-carboxamide) or compound (3-(benzenesulfonyl)-N-(5-isoxazol-5-yl-1,3,4-oxadiazol-2-yl)propanamide) or their pharmaceutically acceptable salts or solvates , which retain the capability of docking into ATP-binding site of phosphofructokinase (PFK-1), for use according to claim 2, wherein the compounds are for use as an inhibitor of PFK-1 enzyme in order to decrease lactate generation by cancer cells.

4. Compound (2-[(5-benzo[1,3]dioxol-5-yl-1,3,4-oxadiazol-2-yl)sulfanyl]-N-isoxazol-3-yl-acetamide), compound (3-methoxy-6-(3-{1-[(5-methyl-1,2,4-oxadiazol-3-yl)methyl]-1H-pyrazol-3-yl}phenyl) pyridazine), compound N-[5-(methanesulfonamido)-1,3,4-thiadiazol-2-yl]-6,7,8,9-tetrahydro-5H-carbazole-3-carboxamide), compound (3-(4-chlorophenyl)sulfonyl-N-(5-isoxazol-5-yl-1,3,4-oxadiazol-2-yl)propanamide, compound (4-[3-(5-amino-1,3,4-oxadiazol-2-yl)isoxazol-5-yl]phenol), compound ((2R)-N-(5-isoxazol-5-yl-1,3,4-oxadiazol-2-yl)-2,3-dihydro-1,4-benzodioxine-2-carboxamide) or compound (3-(benzenesulfonyl)-N-(5-isoxazol-5-yl-1,3,4-oxadiazol-2-yl)propanamide) or their pharmaceutically acceptable salts or solvates for use according to any of the preceding claims, wherein the concentration of the compound is 200 µM or less, preferably in the range from 5 to 100 µM.

5. Compound (2-[(5-benzo[1,3]dioxol-5-yl-1,3,4-oxadiazol-2-yl)sulfanyl]-N-isoxazol-3-yl-acetamide), compound (3-methoxy-6-(3-{1-[(5-methyl-1,2,4-oxadiazol-3-yl)methyl]-1H-pyrazol-3-yl}phenyl) pyridazine), compound N-[5-(methanesulfonamido)-1,3,4-thiadiazol-2-yl]-6,7,8,9-tetrahydro-5H-carbazole-3-carboxamide), compound (3-(4-chlorophenyl)sulfonyl-N-(5-isoxazol-5-yl-1,3,4-oxadiazol-2-yl)propanamide, compound (4-[3-(5-amino-1,3,4-oxadiazol-2-yl)isoxazol-5-yl]phenol), compound ((2R)-N-(5-isoxazol-5-yl-1,3,4-oxadiazol-2-yl)-2,3-dihydro-1,4-benzodioxine-2-carboxamide) or compound (3-(benzenesulfonyl)-N-(5-isoxazol-5-yl-1,3,4-oxadiazol-2-yl)propanamide) or their pharmaceutically acceptable salts or solvates for use according to any of the preceding claims, wherein any of the compounds is used alone or in any combination thereof.

6. Compound (2-[(5-benzo[1,3]dioxol-5-yl-1,3,4-oxadiazol-2-yl)sulfanyl]-N-isoxazol-3-yl-acetamide), compound (3-methoxy-6-(3-{1-[(5-methyl-1,2,4-oxadiazol-3-yl)methyl]-1H-pyrazol-3-yl}phenyl) pyridazine), compound N-[5-(methanesulfonamido)-1,3,4-thiadiazol-2-yl]-6,7,8,9-tetrahydro-5H-carbazole-3-carboxamide), compound (3-(4-chlorophenyl)sulfonyl-N-(5-isoxazol-5-yl-1,3,4-oxadiazol-2-yl)propanamide, compound (4-[3-(5-amino-1,3,4-oxadiazol-2-yl)isoxazol-5-yl]phenol), compound ((2R)-N-(5-isoxazol-5-yl-1,3,4-oxadiazol-2-yl)-2,3-dihydro-1,4-benzodioxine-2-carboxamide) or compound (3-(benzenesulfonyl)-N-(5-isoxazol-5-yl-1,3,4-oxadiazol-2-yl)propanamide) or their pharmaceutically acceptable salts or solvates for use according to any of the preceding claims, wherein any of the compounds is used sequentially in regular time intervals, preferably every 10 to 48 hours, more preferably every 16 to 24 hours.

7. Compound (2-[(5-benzo[1,3]dioxol-5-yl-1,3,4-oxadiazol-2-yl)sulfanyl]-N-isoxazol-3-yl-acetamide), compound (3-methoxy-6-(3-{1-[(5-methyl-1,2,4-oxadiazol-3-yl)methyl]-1H-pyrazol-3-yl}phenyl) pyridazine), compound N-[5-(methanesulfonamido)-1,3,4-thiadiazol-2-yl]-6,7,8,9-tetrahydro-5H-carbazole-3-carboxamide), compound (3-(4-chlorophenyl)sulfonyl-N-(5-isoxazol-5-yl-1,3,4-oxadiazol-2-yl)propanamide, compound (4-[3-(5-amino-1,3,4-oxadiazol-2-yl)isoxazol-5-yl]phenol), compound ((2R)-N-(5-isoxazol-5-yl-1,3,4-oxadiazol-2-yl)-2,3-dihydro-1,4-benzodioxine-2-carboxamide) or compound (3-(benzenesulfonyl)-N-(5-isoxazol-5-yl-1,3,4-oxadiazol-2-yl)propanamide) or their pharmaceutically acceptable salts or solvates for use according to any of the preceding claims, wherein any of the compounds is used with other anticancer treatments and/or medicaments.

8. Compound (2-[(5-benzo[1,3]dioxol-5-yl-1,3,4-oxadiazol-2-yl)sulfanyl]-N-isoxazol-3-yl-acetamide), compound (3-methoxy-6-(3-{1-[(5-methyl-1,2,4-oxadiazol-3-yl)methyl]-1H-pyrazol-3-yl}phenyl) pyridazine), compound N-[5-(methanesulfonamido)-1,3,4-thiadiazol-2-yl]-6,7,8,9-tetrahydro-5H-carbazole-3-carboxamide), compound (3-(4-chlorophenyl)sulfonyl-N-(5-isoxazol-5-yl-1,3,4-oxadiazol-2-yl)propanamide, compound (4-[3-(5-amino-1,3,4-oxadiazol-2-yl)isoxazol-5-yl]phenol), compound ((2R)-N-(5-isoxazol-5-yl-1,3,4-oxadiazol-2-yl)-2,3-dihydro-1,4-benzodioxine-2-carboxamide) or compound (3-(benzenesulfonyl)-N-(5-isoxazol-5-yl-1,3,4-oxadiazol-2-yl)propanamide) or their pharmaceutically acceptable salts or solvates for use according to claim 7, wherein any of the compounds is administered simultaneously or sequentially with other anticancer treatments and/or medicaments.

## Patentansprüche

1. Eine Verbindung, ausgewählt aus der Gruppe, bestehend aus
- (2-[(5-Benzo[1,3]dioxol-5-yl-1,3,4-oxadiazol-2-yl)sulfanyl]-N-isoxazol-3-yl-acetamid),
- (3-Methoxy-6-(3-{1-[(5-methyl-1,2,4-oxadiazol-3-yl)methyl]-1H-pyrazol-3-yl}phenyl)pyridazin),
- N-[5-(Methansulfonamido)-1,3,4-thiadiazol-2-yl]-6,7,8,9-tetrahydro-5H-carbazol-3-carboxamid),
- (3-(4-Chlorphenyl)sulfonyl-N-(5-isoxazol-5-yl-1,3,4-oxadiazol-2-yl)propanamid,
- (4-[3-(5-Amino-1,3,4-oxadiazol-2-yl)isoxazol-5-yl]phenol),
- ((2R)-N-(5-Isoxazol-5-yl-1,3,4-oxadiazol-2-yl)-2,3-dihydro-1,4-benzodioxin-2-carboxamid) und
- (3-(Benzolsulfonyl)-N-(5-isoxazol-5-yl-1,3,4-oxadiazol-2-yl)propanamid)
oder deren pharmazeutisch verträgliche Salze oder Solvate zur Verwendung als Arzneimittel.

2. Verbindung (2-[(5-Benzo[1,3]dioxol-5-yl-1,3,4-oxadiazol-2-yl)sulfanyl]-N-isoxazol-3-yl-acetamid), Verbindung (3-Methoxy-6-(3-{1-[(5-methyl-1,2,4-oxadiazol-3-yl)methyl]-1H-pyrazol-3-yl}phenyl) pyridazin), Verbindung N-[5-(Methansulfonamido)-1,3,4-thiadiazol-2-yl]-6,7,8,9-tetrahydro-5H-carbazol-3-carboxamid), Verbindung (3-(4-Chlorphenyl)sulfonyl -N-(5-isoxazol-5-yl-1,3,4-oxadiazol-2-yl)propanamid, Verbindung (4-[3-(5-Amino-1,3,4-oxadiazol-2-yl)isoxazol-5-yl]phenol), Verbindung ((2R)-N-(5-Isoxazol-5-yl-1,3,4-oxadiazol-2-yl)-2,3-dihydro-1,4-benzodioxin -2-carboxamid) oder Verbindung (3-(Benzolsulfonyl)-N-(5-isoxazol-5-yl-1,3,4-oxadiazol-2-yl)propanamid) oder deren pharmazeutisch verträgliche Salze oder Solvate zur Verwendung nach Anspruch 1 in der Krebstherapie.

3. Verbindung (2-[(5-Benzo[1,3]dioxol-5-yl-1,3,4-oxadiazol-2-yl)sulfanyl]-N-isoxazol-3-yl-acetamid), Verbindung (3-Methoxy-6-(3-{1-[(5-methyl-1,2,4-oxadiazol-3-yl)methyl]-1H-pyrazol-3-yl}phenyl) pyridazin), Verbindung N-[5-(Methansulfonamido)-1,3,4-thiadiazol-2-yl]-6,7,8,9-tetrahydro-5H-carbazol-3-carboxamid), Verbindung (3-(4-Chlorphenyl)sulfonyl -N-(5-isoxazol-5-yl-1,3,4-oxadiazol-2-yl)propanamid, Verbindung (4-[3-(5-Amino-1,3,4-oxadiazol-2-yl)isoxazol-5-yl]phenol), Verbindung ((2R)-N-(5-Isoxazol-5-yl-1,3,4-oxadiazol-2-yl)-2,3-dihydro-1,4-benzodioxin -2-carboxamid) oder Verbindung (3-(Benzolsulfonyl)-N-(5-isoxazol-5-yl-1,3,4-oxadiazol-2-yl)propanamid) oder deren pharmazeutisch verträgliche Salze oder Solvate, welche die Fähigkeit zum Andocken an die ATP-Bindungsstelle der Phosphofructokinase (PFK-1) beibehalten, zur Verwendung nach Anspruch 2, wobei die Verbindungen zur Verwendung als Inhibitor des PFK-1-Enzyms bestimmt sind, um die Laktatbildung durch Krebszellen zu verringern.

4. Verbindung (2-[(5-Benzo[1,3]dioxol-5-yl-1,3,4-oxadiazol-2-yl)sulfanyl]-N-isoxazol-3-yl-acetamid), Verbindung (3-Methoxy-6-(3-{1-[(5-methyl-1,2,4-oxadiazol-3-yl)methyl]-1H-pyrazol-3-yl}phenyl) pyridazin), Verbindung N-[5-(Methansulfonamido)-1,3,4-thiadiazol-2-yl]-6,7,8,9-tetrahydro-5H-carbazol-3-carboxamid), Verbindung (3-(4-Chlorphenyl)sulfonyl -N-(5-isoxazol-5-yl-1,3,4-oxadiazol-2-yl)propanamid, Verbindung (4-[3-(5-Amino-1,3,4-oxadiazol-2-yl)isoxazol-5-yl]phenol), Verbindung ((2R)-N-(5-Isoxazol-5-yl-1,3,4-oxadiazol-2-yl)-2,3-dihydro-1,4-benzodioxin -2-carboxamid) oder Verbindung (3-(Benzolsulfonyl)-N-(5-isoxazol-5-yl-1,3,4-oxadiazol-2-yl)propanamid) oder deren pharmazeutisch verträgliche Salze oder Solvate zur Verwendung nach einem der vorstehenden Ansprüche, wobei die Konzentration der Verbindung 200 µM) oder weniger beträgt, vorzugsweise im Bereich von 5 bis 100 µM.

5. Verbindung (2-[(5-Benzo[1,3]dioxol-5-yl-1,3,4-oxadiazol-2-yl)sulfanyl]-N-isoxazol-3-yl-acetamid), Verbindung (3-Methoxy-6-(3-{1-[(5-methyl-1,2,4-oxadiazol-3-yl)methyl]-1H-pyrazol-3-yl}phenyl) pyridazin), Verbindung N-[5-(Methansulfonamido)-1,3,4-thiadiazol-2-yl]-6,7,8,9-tetrahydro-5H-carbazol-3-carboxamid), Verbindung (3-(4-Chlorphenyl)sulfonyl -N-(5-isoxazol-5-yl-1,3,4-oxadiazol-2-yl)propanamid, Verbindung (4-[3-(5-Amino-1,3,4-oxadiazol-2-yl)isoxazol-5-yl]phenol), Verbindung ((2R)-N-(5-Isoxazol-5-yl-1,3,4-oxadiazol-2-yl)-2,3-dihydro-1,4-benzodioxin -2-carboxamid) oder Verbindung (3-(Benzolsulfonyl)-N-(5-isoxazol-5-yl-1,3,4-oxadiazol-2-yl)propanamid) oder deren pharmazeutisch verträgliche Salze oder Solvate zur Verwendung nach einem der vorstehenden Ansprüche, wobei jede der Verbindungen allein oder in jeder beliebigen Kombination davon verwendet wird.

6. Verbindung (2-[(5-Benzo[1,3]dioxol-5-yl-1,3,4-oxadiazol-2-yl)sulfanyl]-N-isoxazol-3-yl-acetamid), Verbindung (3-Methoxy-6-(3-{1-[(5-methyl-1,2,4-oxadiazol-3-yl)methyl]-1H-pyrazol-3-yl}phenyl) pyridazin), Verbindung N-[5-(Methansulfonamido)-1,3,4-thiadiazol-2-yl]-6,7,8,9-tetrahydro-5H-carbazol-3-carboxamid), Verbindung (3-(4-Chlorphenyl)sulfonyl -N-(5-isoxazol-5-yl-1,3,4-oxadiazol-2-yl)propanamid, Verbindung (4-[3-(5-Amino-1,3,4-oxadiazol-2-yl)isoxazol-5-yl]phenol), Verbindung ((2R)-N-(5-Isoxazol-5-yl-1,3,4-oxadiazol-2-yl)-2,3-dihydro-1,4-benzodioxin -2-carboxamid) oder Verbindung (3-(Benzolsulfonyl)-N-(5-isoxazol-5-yl-1,3,4-oxadiazol-2-yl)propanamid) oder deren pharmazeutisch verträgliche Salze oder Solvate zur Verwendung nach einem der vorstehenden Ansprüche, wobei jede der Verbindungen nacheinander in regelmäßigen Zeitintervallen verwendet wird, vorzugsweise alle 10 bis 48 Stunden, besonders bevorzugt alle 16 bis 24 Stunden.

7. Verbindung (2-[(5-Benzo[1,3]dioxol-5-yl-1,3,4-oxadiazol-2-yl)sulfanyl]-N-isoxazol-3-yl-acetamid), Verbindung (3-Methoxy-6-(3-{1-[(5-methyl-1,2,4-oxadiazol-3-yl)methyl]-1H-pyrazol-3-yl}phenyl) pyridazin), Verbindung N-[5-(Methansulfonamido)-1,3,4-thiadiazol-2-yl]-6,7,8,9-tetrahydro-5H-carbazol-3-carboxamid), Verbindung (3-(4-Chlorphenyl)sulfonyl -N-(5-isoxazol-5-yl-1,3,4-oxadiazol-2-yl)propanamid, Verbindung (4-[3-(5-Amino-1,3,4-oxadiazol-2-yl)isoxazol-5-yl]phenol), Verbindung ((2R)-N-(5-Isoxazol-5-yl-1,3,4-oxadiazol-2-yl)-2,3-dihydro-1,4-benzodioxin -2-carboxamid) oder Verbindung (3-(Benzolsulfonyl)-N-(5-isoxazol-5-yl-1,3,4-oxadiazol-2-yl)propanamid) oder deren pharmazeutisch verträgliche Salze oder Solvate zur Verwendung nach einem der vorstehenden Ansprüche, wobei jede der Verbindungen in Kombination mit anderen Antikrebsbehandlungen und/oder Arzneimitteln verwendet wird.

8. Verbindung (2-[(5-Benzo[1,3]dioxol-5-yl-1,3,4-oxadiazol-2-yl)sulfanyl]-N-isoxazol-3-yl-acetamid), Verbindung (3-Methoxy-6-(3-{1-[(5-methyl-1,2,4-oxadiazol-3-yl)methyl]-1H-pyrazol-3-yl}phenyl) pyridazin), Verbindung N-[5-(Methansulfonamido)-1,3,4-thiadiazol-2-yl]-6,7,8,9-tetrahydro-5H-carbazol-3-carboxamid), Verbindung (3-(4-Chlorphenyl)sulfonyl -N-(5-isoxazol-5-yl-1,3,4-oxadiazol-2-yl)propanamid, Verbindung (4-[3-(5-Amino-1,3,4-oxadiazol-2-yl)isoxazol-5-yl]phenol), Verbindung ((2R)-N-(5-Isoxazol-5-yl-1,3,4-oxadiazol-2-yl)-2,3-dihydro-1,4-benzodioxin -2-carboxamid) oder Verbindung (3-(Benzolsulfonyl)-N-(5-isoxazol-5-yl-1,3,4-oxadiazol-2-yl)propanamid) oder deren pharmazeutisch verträgliche Salze oder Solvate zur Verwendung nach Anspruch 7, wobei jede der Verbindungen gleichzeitig oder nacheinander in Kombination mit anderen Antikrebsbehandlungen und/oder Arzneimitteln verwendet wird.

## Revendications

1. Un composé sélectionné dans le groupe se composant de
- (2-[(5-benzo[1,3]dioxole-5-yl-1,3,4-oxadiazole-2-yl)sulfanyle]-N-isoxazole-3-yl-acétamide),
- (3-méthoxy-6-(3-{1-[(5-méthyl-1,2,4-oxadiazole-3-yl)méthyl]-1H-pyrazole-3-yl}phényl) pyridazine),
- N-[5-(méthanesulfonamide)-1,3,4-thiadiazole-2-yl]-6,7,8,9-tétrahydro-5H-carbazole-3-carboxamide),
- (3-(4-chlorophényl)sulfonyle-N-(5-isoxazole-5-yl-1,3,4-oxadiazole-2-yl)propanamide,
- (4-[3-(5-amino-1,3,4-oxadiazole-2-yl)isoxazole-5-yl]phénol),
- ((2R)-N-(5-isoxazole-5-yl-1,3,4-oxadiazole-2-yl)-2,3-dihydro-1,4-benzodioxine-2-carboxamide) et
- (3-(benzènesulfonyle)-N-(5-isoxazole-5-yl-1,3,4-oxadiazole-2-yl)propanamide)
ou leurs sels ou solvates acceptables d'un point de vue pharmaceutique, pour une utilisation comme médicament.

2. Composé (2-[(5-benzo[1,3]dioxole-5-yl-1,3,4-oxadiazole-2-yl)sulfanyle]-N-isoxazole-3-yl-acétamide), composé (3-méthoxy-6-(3-{1-[(5-méthyl-1,2,4-oxadiazole-3-yl)méthyl]-1H-pyrazole-3-yl}phényl) pyridazine), composé N-[5-(méthanesulfonamide)-1,3,4-thiadiazole-2-yl]-6,7,8,9-tétrahydro-5H-carbazole-3-carboxamide), composé (3-(4-chlorophényl)sulfonyle-N-(5-isoxazole-5-yl-1,3,4-oxadiazole-2-yl)propanamide, composé (4-[3-(5-amino-1,3,4-oxadiazole-2-yl)isoxazole-5-yl]phénol), composé ((2R)-N-(5-isoxazole-5-yl-1,3,4-oxadiazole-2-yl)-2,3-dihydro-1,4-benzodioxine-2-carboxamide) ou composé (3-(benzènesulfonyle)-N-(5-isoxazole-5-yl-1,3,4-oxadiazole-2-yl)propanamide) ou leurs sels ou solvates acceptables d'un point de vue pharmaceutique pour une utilisation selon la revendication 1 dans le traitement du cancer.

3. Composé (2-[(5-benzo[1,3]dioxole-5-yl-1,3,4-oxadiazole-2-yl)sulfanyle]-N-isoxazole-3-yl-acétamide), composé (3-méthoxy-6-(3-{1-[(5-méthyl-1,2,4-oxadiazole-3-yl)méthyl]-1H-pyrazole-3-yl}phényl) pyridazine), composé N-[5-(méthanesulfonamide)-1,3,4-thiadiazole-2-yl]-6,7,8,9-tétrahydro-5H-carbazole-3-carboxamide), composé (3-(4-chlorophényl)sulfonyle-N-(5-isoxazole-5-yl-1,3,4-oxadiazole-2-yl)propanamide, composé (4-[3-(5-amino-1,3,4-oxadiazole-2-yl)isoxazole-5-yl]phénol), composé ((2R)-N-(5-isoxazole-5-yl-1,3,4-oxadiazole-2-yl)-2,3-dihydro-1,4-benzodioxine-2-carboxamide) ou composé (3-(benzènesulfonyle)-N-(5-isoxazole-5-yl-1,3,4-oxadiazole-2-yl)propanamide) ou leurs sels ou solvates acceptables d'un point de vue pharmaceutique, qui conservent l'aptitude à se fixer sur un site de liaison d'ATP de phosphofructokinase (PFK-1), pour une utilisation selon la revendication 2, où les composés sont destinés à une utilisation en tant qu'inhibiteur de l'enzyme PFK-1 pour la réduction de la production de lactate par les cellules cancéreuses.

4. Composé (2-[(5-benzo[1,3]dioxole-5-yl-1,3,4-oxadiazole-2-yl)sulfanyle]-N-isoxazole-3-yl-acétamide), composé (3-méthoxy-6-(3-{1-[(5-méthyl-1,2,4-oxadiazole-3-yl)méthyl]-1H-pyrazole-3-yl}phényl) pyridazine), composé N-[5-(méthanesulfonamide)-1,3,4-thiadiazole-2-yl]-6,7,8,9-tétrahydro-5H-carbazole-3-carboxamide), composé (3-(4-chlorophényl)sulfonyle-N-(5-isoxazole-5-yl-1,3,4-oxadiazole-2-yl)propanamide, composé (4-[3-(5-amino-1,3,4-oxadiazole-2-yl)isoxazole-5-yl]phénol), composé ((2R)-N-(5-isoxazole-5-yl-1,3,4-oxadiazole-2-yl)-2,3-dihydro-1,4-benzodioxine-2-carboxamide) ou composé (3-(benzènesulfonyle)-N-(5-isoxazole-5-yl-1,3,4-oxadiazole-2-yl)propanamide) ou leurs sels ou solvates acceptables d'un point de vue pharmaceutique pour une utilisation selon l'une quelconque des revendications précédentes, où la concentration du composé est de 200 µM) ou moins, de préférence dans une fourchette allant de 5 à 100 µM.

5. Composé (2-[(5-benzo[1,3]dioxole-5-yl-1,3,4-oxadiazole-2-yl)sulfanyle]-N-isoxazole-3-yl-acétamide), composé (3-méthoxy-6-(3-{1-[(5-méthyl-1,2,4-oxadiazole-3-yl)méthyl]-1H-pyrazole-3-yl}phényl) pyridazine), composé N-[5-(méthanesulfonamide)-1,3,4-thiadiazole-2-yl]-6,7,8,9-tétrahydro-5H-carbazole-3-carboxamide), composé (3-(4-chlorophényl)sulfonyle-N-(5-isoxazole-5-yl-1,3,4-oxadiazole-2-yl)propanamide, composé (4-[3-(5-amino-1,3,4-oxadiazole-2-yl)isoxazole-5-yl]phénol), composé ((2R)-N-(5-isoxazole-5-yl-1,3,4-oxadiazole-2-yl)-2,3-dihydro-1,4-benzodioxine-2-carboxamide) ou composé (3-(benzènesulfonyle)-N-(5-isoxazole-5-yl-1,3,4-oxadiazole-2-yl)propanamide) ou leurs sels ou solvates acceptables d'un point de vue pharmaceutique pour une utilisation selon l'une quelconque des revendications précédentes, où l'un quelconque des composés est utilisé seul ou en combinaison avec d'autres composés ci-dessus.

6. Composé (2-[(5-benzo[1,3]dioxole-5-yl-1,3,4-oxadiazole-2-yl)sulfanyle]-N-isoxazole-3-yl-acétamide), composé (3-méthoxy-6-(3-{1-[(5-méthyl-1,2,4-oxadiazole-3-yl)méthyl]-1H-pyrazole-3-yl}phényl) pyridazine), composé N-[5-(méthanesulfonamide)-1,3,4-thiadiazole-2-yl]-6,7,8,9-tétrahydro-5H-carbazole-3-carboxamide), composé (3-(4-chlorophényl)sulfonyle-N-(5-isoxazole-5-yl-1,3,4-oxadiazole-2-yl)propanamide, composé (4-[3-(5-amino-1,3,4-oxadiazole-2-yl)isoxazole-5-yl]phénol), composé ((2R)-N-(5-isoxazole-5-yl-1,3,4-oxadiazole-2-yl)-2,3-dihydro-1,4-benzodioxine-2-carboxamide) ou composé (3-(benzènesulfonyle)-N-(5-isoxazole-5-yl-1,3,4-oxadiazole-2-yl)propanamide) ou leurs sels ou solvates acceptables d'un point de vue pharmaceutique pour une utilisation selon l'une quelconque des revendications précédentes, où l'un quelconque des composés est utilisé de façon séquentielle à des intervalles de temps réguliers, de préférence toutes les 10 à 48 heures, idéalement toutes les 16 à 24 heures.

7. Composé (2-[(5-benzo[1,3]dioxole-5-yl-1,3,4-oxadiazole-2-yl)sulfanyle]-N-isoxazole-3-yl-acétamide), composé (3-méthoxy-6-(3-{1-[(5-méthyl-1,2,4-oxadiazole-3-yl)méthyl]-1H-pyrazole-3-yl}phényl) pyridazine), composé N-[5-(méthanesulfonamide)-1,3,4-thiadiazole-2-yl]-6,7,8,9-tétrahydro-5H-carbazole-3-carboxamide), composé (3-(4-chlorophényl)sulfonyle-N-(5-isoxazole-5-yl-1,3,4-oxadiazole-2-yl)propanamide, composé (4-[3-(5-amino-1,3,4-oxadiazole-2-yl)isoxazole-5-yl]phénol), composé ((2R)-N-(5-isoxazole-5-yl-1,3,4-oxadiazole-2-yl)-2,3-dihydro-1,4-benzodioxine-2-carboxamide) ou composé (3-(benzènesulfonyle)-N-(5-isoxazole-5-yl-1,3,4-oxadiazole-2-yl)propanamide) ou leurs sels ou solvates acceptables d'un point de vue pharmaceutique pour une utilisation selon l'une quelconque des revendications précédentes, où l'un quelconque des composés est utilisé avec d'autres traitements et/ou médicaments anticancéreux.

8. Composé (2-[(5-benzo[1,3]dioxole-5-yl-1,3,4-oxadiazole-2-yl)sulfanyle]-N-isoxazole-3-yl-acétamide), composé (3-méthoxy-6-(3-{1-[(5-méthyl-1,2,4-oxadiazole-3-yl)méthyl]-1H-pyrazole-3-yl}phényl) pyridazine), composé N-[5-(méthanesulfonamide)-1,3,4-thiadiazole-2-yl]-6,7,8,9-tétrahydro-5H-carbazole-3-carboxamide), composé (3-(4-chlorophényl)sulfonyle-N-(5-isoxazole-5-yl-1,3,4-oxadiazole-2-yl)propanamide, composé (4-[3-(5-amino-1,3,4-oxadiazole-2-yl)isoxazole-5-yl]phénol), composé ((2R)-N-(5-isoxazole-5-yl-1,3,4-oxadiazole-2-yl)-2,3-dihydro-1,4-benzodioxine-2-carboxamide) ou composé (3-(benzènesulfonyle)-N-(5-isoxazole-5-yl-1,3,4-oxadiazole-2-yl)propanamide) ou leurs sels ou solvates acceptables d'un point de vue pharmaceutique pour une utilisation selon la revendication 7, où l'un quelconque des composés est administré simultanément ou de façon séquentielle avec d'autres traitements et/ou médicaments anticancéreux.
